# EUROPÄISCHE PATENTSCHRIFT

(11) **EP 3 073 922 B1**
(45) Veröffentlichungstag und Bekanntmachung des Hinweises auf die Patenterteilung: **14.02.2018**
(21) Anmeldenummer: 14723747.3
(22) Anmeldetag: 25.04.2014
(51) Int. Cl.: A61B 5/15, A61B 5/157

(54) **EINZELVERPACKTE EINWEG-BLUTTESTEINHEIT**
INDIVIDUALLY PACKAGED DISPOSABLE BLOOD TESTING UNIT
UNITÉ DE TEST SANGUIN À USAGE UNIQUE EN EMBALLAGE INDIVIDUEL

(30) Priorität: 26.04.2013 AT 502902013
(43) Veröffentlichungstag der Anmeldung: 05.10.2016
(73) Patentinhaber: Polymer Technology Systems, Inc., Indianapolis, IN 46268 (US)
(72) Erfinder: HAFELLNER, Reinhard, A-8724 Spielberg (AT); RITTMANNSBERGER, Franz, A-8720 Knittelfeld (AT)
(74) Vertreter: Zinkler, Franz
(86) Internationale Anmeldenummer: PCT/EP2014/058497
(87) Internationale Veröffentlichungsnummer: WO 2014/174096

(56) Entgegenhaltungen:
- EP-A1- 1 293 222
- EP-A1- 1 529 488
- DE-B1- 2 803 345
- US-B2- 7 846 110

## Beschreibung

Die Erfindung betrifft ein Einweg-Produkt zum einmaligen Durchführen eines Bluttests durch einen Benutzer, eine Test-Anordnung und ein Verfahren zum Herstellen eines Einweg-Produkts zum einmaligen Durchführen eines Bluttests durch einen Benutzer.

Diabetiker müssen in der Regel mehrmals täglich Messungen ihres Blutzuckerspiegels durchführen, um zum Beispiel zu ermitteln, ob - und falls ja in welcher Menge - das Zuführen von Insulin oder oraler Medikation erforderlich ist. Herkömmlich verwenden Diabetiker hierfür Stechnadeln, mit denen sie eine Blutprobe entnehmen. Die entnommene Blutmenge wird dann auf einen separaten Teststreifen aufgebracht, mit dem das entnommene Blut chemisch wechselwirkt, womit ein Reaktionsprodukt entsteht. Mit einem Messgerät, welches den Teststreifen vermisst, wird dann eine Glukosemessung durchgeführt. Bei einem solchen System muss ein Benutzer viele unterschiedliche Teile handhaben und mit sich führen. Ferner treten mitunter Hygieneprobleme auf, welche aus einer mangelnden Sterilität der einzelnen Komponenten, insbesondere durch die Handhabung der Komponenten, resultieren.

US 7,846,110 offenbart eine integrale Bluttesteinheit, bei der die Elemente der Stechhilfe sowie der Testsubstanz zum Wechselwirken mit einer entnommenen Blutprobe in einem Bauteil integriert sind. Eine solche integrale Bluttesteinheit ist auf ein Detektorgerät aufzusetzen, um das Reaktionsprodukt zwischen der Blutprobe und der Testsubstanz sensorisch auszulesen.

Vor einer Benutzung einer Bluttesteinheit kann es vorkommen, dass diese längere Zeit gelagert wird. Während dieser Lagerzeit muss darauf geachtet werden, dass sich die Funktionsfähigkeit der Bluttesteinheit nicht durch Umwelteinflüsse verschlechtert. Die Haltbarkeit von herkömmlichen Bluttesteinheiten ist für viele Fälle nicht ausreichend lang. Außerdem kann es passieren, dass herkömmliche Bluttesteinheiten verunreinigt sind und somit bei einem Benutzer Infektionen oder sonstige medizinische Schäden hervorrufen.

Es ist eine Aufgabe der vorliegenden Erfindung, eine von einem Benutzer einfach handhabbare Bluttesteinheit bereitzustellen, die eine hohe Haltbarkeit aufweist und einen Benutzer zuverlässig vor Infektionen schützt.

Diese Aufgabe wird durch die Gegenstände mit den Merkmalen gemäß den unabhängigen Patentansprüchen gelöst. Weitere Ausführungsbeispiele sind in den abhängigen Ansprüchen gezeigt.

Gemäß einem Ausführungsbeispiel der vorliegenden Erfindung ist ein Einweg-Produkt zum (genau oder nur) einmaligen Durchführen eines Bluttests durch einen Benutzer geschaffen, wobei das Einweg-Produkt eine integrale Bluttesteinheit mit einer Stechhilfe zum Einstechen in ein Körperteil des Benutzers zum Entnehmen einer Blutprobe und mit einer Testsubstanz zum Wechselwirken mit der entnommenen Blutprobe zum Erzeugen eines detektierbaren Indikators für eine Eigenschaft der Blutprobe, wenn die Bluttesteinheit mit einem (separat von der integralen Bluttesteinheit vorgesehenen und mit dieser zum Detektieren des Ergebnisses des durchgeführten Bluttests funktionell und strukturell zusammenwirkenden) Detektorgerät gekoppelt ist (zum Beispiel darauf aufgesteckt ist), und eine Einzelverpackung (wie zum Beispiel eine Folienumhüllung oder eine andere vorzugsweise flexible Umhüllung) als Feuchtigkeitsbarriere aufweist, welche Einzelverpackung zumindest einen Teil der Bluttesteinheit gegenüber der Umgebung hermetisch umschließt (d.h. teil umfänglich oder allseitig feuchtigkeitsundurchlässig umgibt) und die von einem Benutzer händisch (d.h. bloß mittels der Hände des Benutzers und somit werkzeugfrei) öffenbar ist (insbesondere unter irreversibler Zerstörung der Einzelverpackung aufgerissen werden kann), wobei die Einzelverpackung eine Mehrschichtfolie aufweist, die eine Feuchtigkeitsdiffusionsbarrierenschicht enthält, deren eine Oberfläche mit einer heißversiegelbaren Schicht bedeckt ist und deren gegenüberliegende andere Oberfläche mit einer Schutzschicht zum Schutz der Feuchtigkeitsdiffusionsbarrierenschicht bedeckt ist, wobei die Einzelverpackung einen starren Grundkörper, insbesondere ausgebildet als Spritzgussteil, aufweist, der mit der Mehrschichtfolie verschlossen ist, und wobei der starre Grundkörper becherförmig ist und eine Öffnung aufweist, die von der Mehrschichtfolie ringförmig umschlossen ist. Gemäß einem weiteren Ausführungsbeispiel der vorliegenden Erfindung ist eine Test-Anordnung bereitgestellt, die ein Einweg-Produkt mit den oben beschriebenen Merkmalen zum (insbesondere genau oder nur) einmaligen Durchführen eines Bluttests durch einen Benutzer, und ein (insbesondere darauf angepasstes) Detektorgerät aufweist, mit dem die Bluttesteinheit derart koppelbar ist, dass im gekoppelten Zustand die Stechhilfe der Bluttesteinheit in ein Körperteil des Benutzers zum Entnehmen einer Blutprobe einstechbar ist und die entnommene Blutprobe mit der Testsubstanz der Bluttesteinheit zum Erzeugen eines Indikators für eine Eigenschaft der Blutprobe in Wechselwirkung bringbar ist, welcher Indikator mittels des Detektorgeräts detektierbar ist (d.h., dass das Detektorgerät konfiguriert sein kann, den Indikator qualitativ oder sogar quantitativ zu ermitteln und somit das Ergebnis des mittels der Bluttesteinheit durchgeführten Bluttests sensorisch zu erfassen).

Gemäß einem weiteren Ausführungsbeispiel der vorliegenden Erfindung ist ein Verfahren zum Herstellen eines Einweg-Produkts zum einmaligen Durchführen eines Bluttests durch einen Benutzer geschaffen, wobei bei dem Verfahren eine integrale Bluttesteinheit mit einer Stechhilfe zum Einstechen in ein Körperteil (insbesondere einen Finger) des Benutzers zum Entnehmen einer Blutprobe und mit einer Testsubstanz zum Wechselwirken mit der entnommenen Blutprobe zum Erzeugen eines für eine Eigenschaft der Blutprobe indikativen Indikators, der detektierbar ist, wenn die Bluttesteinheit mit einem Detektorgerät gekoppelt ist, bereitgestellt wird, und zumindest ein Teil der Bluttesteinheit gegenüber einer Umgebung mit einer Einzelverpackung als Feuchtigkeitsbarriere derart hermetisch verschlossen (insbesondere derart teilumfänglich oder vollumfänglich ummantelt oder umhüllt) wird, dass die Einzelverpackung von einem Benutzer händisch öffenbar ist, wobei die Einzelverpackung eine Mehrschichtfolie aufweist, die eine Feuchtigkeitsdiffusionsbarrierenschicht enthält, deren eine Oberfläche mit einer heißversiegelbaren Schicht bedeckt ist und deren gegenüberliegende andere Oberfläche mit einer Schutzschicht zum Schutz der Feuchtigkeitsdiffusionsbarrierenschicht bedeckt ist, wobei die Einzelverpackung einen starren Grundkörper, insbesondere ausgebildet als Spritzgussteil, aufweist, der mit der Mehrschichtfolie verschlossen ist, und wobei der starre Grundkörper becherförmig ist und eine Öffnung aufweist, die von der Mehrschichtfolie ringförmig umschlossen ist. Im Rahmen dieser Patentanmeldung wird unter dem Begriff "Einweg-Produkt" insbesondere ein Produkt verstanden, dass für den einmaligen Gebrauch ausgebildet ist und darauf beschränkt ist. Durch den erstmaligen Gebrauch kann das Einweg-Produkt somit derart irreversibel verändert werden, dass ein nochmaliger Gebrauch verunmöglicht wird.

Im Rahmen dieser Patentanmeldung wird unter dem Begriff "integrale Bluttesteinheit" insbesondere eine Bluttesteinheit verstanden, die als einstückiger Körper zur Verwendung mit einem korrespondierend ausgebildeten Detektorgerät ausgebildet ist. Mit solch einer integralen Bluttesteinheit können alle für einen Bluttest, insbesondere eine Messung des Blutzuckerspiegels, erforderlichen Komponenten, mit Ausnahme des Detektorgeräts zum Auswerten des Ergebnisses des Bluttests, in der integralen Bluttesteinheit vereinigt sein. Eine solche integrale Bluttesteinheit kann somit angefangen von dem Auslösen eines Bluttests durch Erkennen einer Druckbeaufschlagung durch ein Körperteil des Benutzers, über das Einstechen einer Stechhilfe in ein Körperteil des Benutzers zum Entnehmen einer Blutprobe, über das Führen der entnommenen Blutprobe durch eine Kapillare zu einer Testsubstanz bis hin zum Herstellen einer Wechselwirkung der Testsubstanz mit der Blutprobe alle Funktionen und Prozesse des Bluttests übernehmen. Somit hat ein Benutzer bei einer integralen Bluttesteinheit nur eine einzige Komponente zu handhaben und zusammenwirkend mit dem Detektorgerät zu verwenden.

Im Rahmen dieser Patentanmeldung wird unter dem Begriff "Indikator für eine Eigenschaft der Blutprobe" insbesondere eine messtechnisch erfassbare Substanz oder ein messtechnisch erfassbarer Parameter verstanden, die oder der von dem Detektorgerät erfasst werden kann, wenn die Testsubstanz der integralen Bluttesteinheit mit der Blutprobe in Kontakt gerät. Als Ergebnis einer solchen Wechselwirkung kann eine chemische Reaktion stattfinden, deren Reaktionsprodukt als Indikator zum Beispiel durch eine optische Reflexions-Absorptions- oder Fluoreszenzmessung nachgewiesen werden kann. Aus dem Messsignal kann der Indikator dann qualitativ oder sogar quantitativ ermittelt werden.

Gemäß einem exemplarischen Ausführungsbeispiel der Erfindung ist durch das Unterbringen der integralen Bluttesteinheit, die auch die zum Beispiel trockenchemische Testsubstanz enthält, innerhalb der nach außen hin hermetisch abdichtenden Einzelverpackung somit zuverlässig unterdrückt, dass selbst bei langen Lagerzeiten des Produkts größere Mengen (insbesondere wässrige) Feuchtigkeit aus der Umgebung unerwünscht mit der Testsubstanz chemisch reagieren, womit die spätere Verwendung des Produkts zum Durchführen eines Bluttests negativ beeinflusst oder sogar unmöglich gemacht würde. Auch kann das Eindringen von Keimen durch die Einzelverpackung hindurch zu der Bluttesteinheit zuverlässig unterdrückt werden, so dass auch über einen längeren Zeitraum hinweg gerade die Einstechhilfe im Inneren der Einzelverpackung keimfrei gehalten werden kann. Indem immer nur genau eine integrale Bluttesteinheit innerhalb der Einzelverpackung untergebracht ist, kann auch vermieden werden, dass ein Benutzer eine Bluttesteinheit entnimmt und eine oder mehrere andere Bluttesteinheiten dadurch hinsichtlich deren Sterilität bzw. Feuchtigkeitsabschirmung negativ beeinflusst werden. Das Material der Einzelverpackung selbst dient dabei als Flüssigkeits- bzw. Feuchtigkeitsbarriere, die das Eindringen von Flüssigkeit bzw. Feuchtigkeit in das Innere des Einwegprodukts zumindest stark unterdrückt.

Im Weiteren werden zusätzliche exemplarische Ausführungsbeispiele des Einweg-Produkts, der Test-Anordnung und des Verfahrens beschrieben.

Gemäß einem exemplarischen Ausführungsbeispiel ist die Einzelverpackung flüssigkeitsdicht und somit für Flüssigkeiten im Wesentlichen bzw. sogar vollständig undurchdringbar bzw. impermeabel. Der Fachmann wird allerdings erkennen, dass eine äußerst geringe Restdurchlässigkeit einer Einzelverpackung für jegliche Form von Flüssigkeiten oder Feuchtigkeit in vielen Fällen nicht vermeidbar sein wird, die allerdings so gering sein sollte, dass es nur in unwesentlichem Umfang zu einem Ansammeln von Feuchtigkeit im Inneren der Einzelverpackung kommen wird. Bevorzugt kann die Einzelverpackung luftfeuchtigkeitsdicht sein. Im Rahmen dieser Patentanmeldung wird unter dem Begriff "luftfeuchtigkeitsdicht" insbesondere verstanden, dass die Wandung der Einzelverpackung durchweg so konfiguriert ist, dass das Durchdringen von Luftfeuchtigkeit und erst recht einer Flüssigkeit, insbesondere von Wasser, durch diese Wandung hindurch im Wesentlichen unterbunden ist. Eine Ursache für das Eindringen einer Flüssigkeit in das Innere der Einzelverpackung ist in vielen Fällen nicht direkter Flüssigkeitskontakt, sondern oftmals die umgebende Luftfeuchtigkeit (zum Beispiel Wasser im meist gasförmigen Zustand). Die Wandung sollte somit "diffusionsdicht" bzw. "dicht gegenüber Luftfeuchtigkeit" oder ähnlichem sein. Insbesondere kann die Wandung vollkommen gasdicht ausgebildet sein, da auch andere Gase für die Bluttesteinheit störend sein können. In einer besonders bevorzugten Ausführungsform ist die Einzelverpackung sogar gasdicht, das heißt undurchlässig bzw. impermeabel für Gase.

Zum Beispiel kann das Material der Einzelverpackung so ausgewählt werden, dass dessen Wasserdampfdurchlässigkeit kleiner als 100 g/(m²*d) ist, insbesondere kleiner als 10 g/(m²*d) ist, wobei "d" für Tag steht. Die Sauerstoffdurchlässigkeit des Materials der Einzelverpackung kann kleiner als 10000 cm/(m²*bar*d) sein, insbesondere kleiner als 1000 cm/(m²*bar*d).

Gemäß einem exemplarischen Ausführungsbeispiel der Erfindung hat die Bluttesteinheit eine Form, deren Dimensionen sich entlang dreier zueinander senkrechter Raumachsen von einem Mittelwert jeweils um weniger als 50 %, insbesondere um weniger als 20 % unterscheiden. Anders ausgedrückt kann in drei zueinander senkrechten Raumrichtungen die Bluttesteinheit eine Länge, eine Breite und eine Höhe aufweisen, aus denen sich ein Mittelwert berechnen lässt. Sowohl die Länge als auch die Breite als auch die Höhe unterscheidet sich von diesem Mittelwert vorzugsweise jeweils um weniger als 50 %, insbesondere um weniger als 20 %. Dadurch ist eine in allen Raumrichtungen kompakte Bluttesteinheit geschaffen, die zum Beispiel im Wesentlichen zylindrisch, im Wesentlichen kugelförmig, im Wesentlichen halbkugelförmig oder im Wesentlichen quader- oder würfelförmig sein kann. Eine derart geformte Bluttesteinheit ist auch besonders platzsparend in einer Einwegverpackung unterbringbar und von einem Benutzer darin einfach handhabbar.

Gemäß der Erfindung weist die Einzelverpackung eine Mehrschichtfolie (insbesondere aufgebaut aus genau drei oder mehr als drei Einzelschichten) auf, die eine Feuchtigkeitsdiffusionsbarrierenschicht enthält, deren eine Oberfläche (insbesondere die innerseitige Oberfläche) mit einer heißversiegelbaren Schicht bedeckt ist und deren gegenüberliegende andere Oberfläche (insbesondere die außenseitige Oberfläche) mit einer Schutzschicht zum Schutz der Feuchtigkeitsdiffusionsbarrierenschicht bedeckt ist. Der Einsatz einer solchen Verbundfolie bietet den Vorteil, dass die häufig empfindliche Feuchtigkeitsdiffusionsbarrierenschicht beidseitig bedeckt wird und somit vor einer Schädigung geschützt wird. Die Feuchtigkeitsdiffusionsbarrierenschicht selbst hat dabei die Aufgabe, die Diffusion von Feuchtigkeit sowie wässriger oder sonstiger flüssiger Substanzen durch die Mehrschichtfolie hindurch zu hemmen oder sogar zu unterbinden. Die außenseitige Schutzschicht schützt einerseits die Feuchtigkeitsdiffusionsbarrierenschicht (zum Beispiel vor chemischer Veränderung, Materialabrieb, Bruchbildung oder Rissbildung) und kann andererseits frei gewählt werden, um weitere Anforderungen der Einzelverpackung (wie zum Beispiel Beschriftbarkeit) zu gewährleisten. Die heißversiegelbare Schicht erlaubt es, eine plane Mehrschichtfolie so zu versiegeln bzw. zu verschweißen, dass dadurch eine allseitig hermetische Abdichtung von deren Innenraum gegenüber der Umgebung erreicht werden kann. Eine derartige dreischichtige Mehrschichtfolie ist leicht, kostengünstig in der Herstellung und bietet dennoch alle Eigenschaften, die für das Einweg-Produkt erforderlich sind.

Gemäß einem Ausführungsbeispiel kann die Einzelverpackung ausschließlich aus der Mehrschichtfolie bestehen. Bei einer solchen Ausführungsform sind außer der Mehrschichtfolie keine weiteren Komponenten erforderlich, um die Einzelverpackung auszubilden, was eine leichtgewichtige und kostengünstige Herstellung ermöglicht.

Gemäß der Erfindung weist die Einzelverpackung einen starren Grundkörper (der zum Beispiel als kostengünstig fertigbares Spritzgussteil ausgebildet sein kann) auf, der mit der Mehrschichtfolie verschlossen ist. Daher können erfindungsgemäß Verpackungen bereitgestellt werden, die nur teilweise aus Mehrschichtfolie und teilweise aus Spritzguss-Komponenten (zum Beispiel der Testeinheit) bestehen. Der starre Grundkörper ist becherförmig (und geformt, um die Bluttesteinheit aufzunehmen) und weist eine Öffnung auf, die von der Mehrschichtfolie ringförmig umschlossen ist. Ein Benutzer braucht dann bloß die Mehrschichtfolie von dem starrem Grundkörper abzulösen, an dem die Mehrschichtfolie zum Beispiel heißversiegelt befestigt sein kann, um die Bluttesteinheit zu entnehmen.

Gemäß einem Ausführungsbeispiel kann die luftfeuchtigkeitsdichte Einzelverpackung nur genau einen Teil der Bluttesteinheit umschließen und kann ein verbleibender Teil der Bluttesteinheit gegenüber der Umgebung unbedeckt bleiben und ebenfalls luftfeuchtigkeitsdicht ausgebildet sein. Ein Ausführungsbeispiel ist somit, dass ein Teil der Verpackung zum Beispiel durch die Mehrschichtfolie gebildet wird, und ein anderer Teil durch ein Bauteil der Testeinheit. Somit kann bei dem Produkt die Sperrschicht teilweise aus der Folie, teilweise aus integralen Bestandteilen der Testeinheit bestehen.

Eine Dicke der Feuchtigkeitsdiffusionsbarrierenschicht kann zum Beispiel in einem Bereich zwischen 2 µm und 20 µm, insbesondere in einem Bereich zwischen 7 µm und 15 µm, liegen. Eine Dicke der heißversiegelbaren Schicht kann zum Beispiel in einem Bereich zwischen 10 µm und 80 µm, insbesondere in einem Bereich zwischen 20 µm und 50 µm, liegen. Eine Dicke der Schutzschicht kann zum Beispiel in einem Bereich zwischen 10 µm und 100 µm, insbesondere in einem Bereich zwischen 30 µm und 50 µm, liegen.

Gemäß einem Ausführungsbeispiel kann die Feuchtigkeitsdiffusionsbarrierenschicht ein Metall (insbesondere Aluminium oder Nickel), ein Metalloxid, Ethylenvinylacetat oder ein Silikat aufweisen oder daraus bestehen. Besonders bevorzugt ist Aluminium, da dieses eine besonders gute Eignung als Diffusionssperre aufweist. Allerdings neigt Aluminium zu Riss- oder Bruchbildung sowie zu Staubablagerungen, so dass es besonders vorteilhaft ist, Aluminium vorzugsweise beidseitig abzudecken.

Gemäß einem Ausführungsbeispiel kann die heißversiegelbare Schicht einen heißversiegelbaren Kunststoff, insbesondere Polyethylen oder Polypropylen, aufweisen oder daraus bestehen. Besonders Polyethylen ist hinsichtlich der Verschweißbarkeitseigenschaften vorteilhaft.

Gemäß einem Ausführungsbeispiel kann die Schutzschicht Polyamid, Polyethylenterephthalat (PET), eine Polyethylenterephthalat-Papier Struktur oder Papier aufweisen oder daraus bestehen. PET hat die Eigenschaft einer guten Bedruckbarkeit. Wird Papier an der Oberfläche verwendet, so kann das Papier mit einer Klebeschicht an der benachbarten Schicht befestigt sein.

Es versteht sich von selbst, dass die Mehrschichtfolie optional eine oder mehrere weitere Schichten aufweisen kann, zum Beispiel Haftvermittlungsschichten Eine Gesamtdicke der Mehrschichtfolie kann zum Beispiel in einem Bereich zwischen 50 µm und 200 µm liegen.

Gemäß einem Ausführungsbeispiel kann das Produkt ein Trocknungsmittel aufweisen, das in Fluidverbindung mit der Bluttesteinheit angeordnet ist. Eventuell durch die als Flüssigkeitsbarriere wirkende Einzelverpackung hindurchgelangende Feuchtigkeit kann somit durch das Trocknungsmittel gebunden werden und so vor einer unerwünschten Wechselwirkung mit der Trockenchemie der Testsubstanz bewahrt werden.

Gemäß einem Ausführungsbeispiel kann das Trocknungsmittel im Inneren der als Feuchtigkeitsbarriere wirkenden Einzelverpackung angeordnet sein. Das Vorsehen von Trocknungsmittel im Inneren der Einzelverpackung sorgt dafür, dass selbst beim Diffundieren geringer Mengen von Feuchtigkeit durch die Einzelverpackung hindurch die Testsubstanz der integralen Bluttesteinheit weiterhin zuverlässig vor einer unerwünschten Reaktion mit der Feuchtigkeit geschützt ist. Das Trocknungsmittel sollte daher vorzugsweise so konfiguriert sein, dass es stark hygroskopische Eigenschaften hat und somit Wasser und andere Fluide zuverlässig von der Testsubstanz der integralen Bluttesteinheit fernhält. Dadurch können die Lagerzeiten des Produkts weiter erhöht werden.

Gemäß einem anderen Ausführungsbeispiel kann das Trocknungsmittel in der als Feuchtigkeitsbarriere wirkenden Einzelverpackung eingebettet sein. Das Trocknungsmittel kann somit auch in dem Material der Einzelverpackung selbst integriert sein. Wenn zum Beispiel die Einzelverpackung als Mehrschichtfolie ausgebildet ist, kann das Trocknungsmittel in oder als Teil einer beidseitig bedeckten Schicht der Mehrschichtfolie vorgesehen sein. Dann kann Flüssigkeit bereits während des Durchdringens der Einzelverpackung gebunden werden und gelangt somit gar nicht erst ins Innere der Einzelverpackung.

Gemäß einem Ausführungsbeispiel kann das Trocknungsmittel ein Molekularsieb, Silikagel und/oder Tonerde aufweisen. Molekularsieb ist die Bezeichnung für natürliche und synthetische Zeolithe, die ein starkes Adsorptionsvermögen für Dämpfe und gelöste Stoffe mit bestimmten Molekülgrößen haben. Molekularsiebe weisen eine große innere Oberfläche (zum Beispiel 500 m²/g bis 1000 m²/g) auf und haben einheitliche Porendurchmesser, die in der Größenordnung der Durchmesser von Molekülen liegen. Kieselgel oder Silikagel ist ein amorphes Siliziumdioxid von gelartiger, gummiartiger bis fester Konsistenz und ist farblos. Es besitzt eine große innere Oberfläche, ist stark hygroskopisch (wasseranziehend) und eignet sich daher als Trockenmittel zum Abschirmen der Bluttesteinheit gegenüber Feuchtigkeit. Tonerde ist eine hygroskopische Form von Aluminiumoxid. Allgemeiner ausgedrückt kann das Trocknungsmittel aus einem Material gebildet sein, das Feuchtigkeit anzieht und in der Regel eine hohe innere poröse Oberfläche besitzt.

Gemäß einem Ausführungsbeispiel kann das Trocknungsmittel, insbesondere in Form von Granulat oder Pulver, in einem feuchtigkeitsdurchlässigen und trocknungsmittelundurchlässigen Sack im Inneren der luftfeuchtigkeitsdichten Einzelverpackung angeordnet sein. Der Sack kann also Feuchtigkeit passieren lassen, aber für Partikel des Trocknungsmittels undurchdringlich sein. Der Sack kann zum Beispiel aus einem Gewebe mit größeren Durchgangslöchern gebildet sein. Ein einziges Kompartment oder Innenvolumen, das durch die Einzelverpackung begrenzt wird, enthält bei dieser Ausführungsform sowohl die Bluttesteinheit als auch das Trocknungsmittel, so dass diese beiden Komponenten in enger Verbindung miteinander stehen.

Gemäß einem Ausführungsbeispiel kann das Trocknungsmittel, insbesondere in Form eines einzigen zusammenhängenden Körpers (möglich sind alternativ aber auch mehrere getrennte Körper), in einem separaten Kompartment der luftfeuchtigkeitsdichten Einzelverpackung angeordnet sein, das mit der Bluttesteinheit, insbesondere durch ein Durchgangsloch in einer Wand des Kompartments, in Fluidverbindung gebracht sein kann. Zum Beispiel kann eine einzige ausreichend große Kugel (mit einem Durchmesser von zum Beispiel zwischen 3 mm und 10 mm) aus einem Trocknungsmittel (zum Beispiel einem der oben genannten Trocknungsmittel) ausreichend sein, um in dem separaten Kompartment untergebracht eine Trocknungsfunktion zu erfüllen, welche die Testsubstanz der integralen Bluttesteinheit vor Feuchtigkeit schützt. Der Trocknungskörper kann über ein Loch in einer Zwischenwandung im Inneren der Einzelverpackung mit der Bluttesteinheit eine Feuchtigkeitskopplung geeigneter Stärke bewirken.

Gemäß einem Ausführungsbeispiel kann das Trocknungsmittel (zum Beispiel eines der oben genannten Trocknungsmittel), insbesondere in Form von in einer porösen Matrix verteilten Trocknungsmittelpartikeln, als flächige Struktur auf einer Innenwand der luftfeuchtigkeitsdichten Einzelverpackung befestigt sein, insbesondere laminiert oder aufgeklebt sein. Zum Beispiel kann das Trocknungsmittel in Form eines Streifens an der Innenseite einer Wandung der Einzelverpackung angebracht sein. Das Befestigen eines solchen Streifens an der Innenwandung der Einzelverpackung kann zum Beispiel mittels Laminierens erfolgen, d.h. mittels Erhitzens der Einzelverpackung bzw. des Streifens und Anhaften des Streifens an der Einzelverpackung im erhitzten Zustand. Durch nachfolgendes Abkühlen kann dann eine feste Verbindung zwischen Streifen und Innenwand ausgebildet werden. Alternativ kann ein solcher Trocknungsstreifen mittels Klebstoff auch an der Innenwand der Einzelverpackung angeklebt werden.

Gemäß einem Ausführungsbeispiel kann das Trocknungsmittel an und/oder in der Bluttesteinheit mechanisch verankert sein oder ein Trockenmittelcompound als struktureller Bestandteil der Bluttesteinheit vorgesehen sein. Das Trockenmittel kann auch in der Testeinheit fixiert sein, zum Beispiel kann ein Kügelchen formschlüssig mit der Testeinheit verbunden sein. Das Trockencompound kann im Spritzgießen verarbeitet werden. Dann kann es auch integraler, struktureller Bestandteil der Testeinheit sein.

Zum Beispiel kann die poröse Matrix aus einem Kunststoff gebildet sein, der sich mit dem Material der Trocknungsmittelpartikel nicht oder nur sehr schlecht vermischt. Dadurch bildet sich ein Kompositmaterial, bei dem die Partikel in der Kunststoffmatrix eingebettet sind, aufgrund der schlechten Vermischbarkeit sich aber feuchtigkeitsdurchlässige Poren oder Kanäle bilden. Diese Kanäle oder Poren bilden eine Fluidverbindung zwischen von dem Trocknungsmittel zu bindender Feuchtigkeit und den Partikeln. Dadurch kann eine verzögerte Wirkung des Trocknungsmittels erreicht werden und somit eine Funktionstüchtigkeit des Trocknungsmittels über einen langen Zeitraum hinweg.

Gemäß einem Ausführungsbeispiel kann bei dem Einweg-Produkt innerhalb der Einzelverpackung nur genau eine Bluttesteinheit enthalten sein. Dadurch ist ausgeschlossen, dass nach Entnahme einer ersten Bluttesteinheit eine oder mehrere andere Bluttesteinheiten an Sterilität und Feuchtigkeitsschutz durch die dann irreversibel zerstörte Einzelverpackung verlieren. Insbesondere kann die Testsubstanz und/oder kann die Stechhilfe der Bluttesteinheit so bemessen sein, dass diese nur für genau einen Bluttest ausreicht bzw. ausreichen. Hinsichtlich der Testsubstanz kann dies bedeuten, dass bei dem ersten Bluttests diese durch Wechselwirkung mit dem Blut so beeinflusst wird, dass sie für einen zweiten Bluttest nicht mehr verwendbar ist. Hinsichtlich der Stechhilfe kann dies bedeuten, dass diese nach dem ersten Bluttests so modifiziert wird, dass ein Durchführen eines weiteren Bluttests mit dieser Stechhilfe nicht mehr möglich ist (zum Beispiel weil diese nach dem ersten Bluttest unverwendbar in ein Gehäuseinneres der Bluttesteinheit zurückgezogen wird).

Gemäß einem Ausführungsbeispiel kann die Bluttesteinheit ausgebildet sein, dass bei dieser nach erstmaligem Einsatz der Stechhilfe ein nochmaliger Einsatz der Stechhilfe verunmöglicht ist. Der Einwegcharakter der luftfeuchtigkeitsdichten Einzelverpackung, die ausgebildet sein kann, nach dem Öffnen durch einen Benutzer nicht mehr wiederverschließbar zu sein, kann somit synergistisch mit einer nur einmaligen Verwendbarkeit der integralen Bluttesteinheit kombiniert werden. Zum Beispiel kann nach dem ersten Verwenden der Stechhilfe diese so in das Innere der Bluttesteinheit zurückgezogen werden, dass ein nochmaliges Verwenden der Stechhilfe funktional ausgeschlossen ist. Somit verhindert ein Doppelschutz hinsichtlich Verpackung und Inhalt ein die Sterilität und Messgenauigkeit tangierendes Mehrfachverwenden der Bluttesteinheit.

Gemäß einem Ausführungsbeispiel kann die Testsubstanz eine trockenchemische, durch Feuchtigkeit veränderliche Substanz aufweisen. Die Testsubstanz kann ausgebildet sein, bei Anwesenheit von Blut mit der darin befindlichen Glukose so zu reagieren, dass ein Reaktionsprodukts optisch erfassbar ist. Die optische Erfassung der Menge des Reaktionsprodukts lässt dann einen Rückschluss auf die Glukosekonzentration in der Blutprobe zu. Derartige Testsubstanzen zum Bestimmen der Glukosekonzentration in Blut können trockenchemisch realisiert sein. Kommen diese vor Durchführung der Glukosemessung mit Luftfeuchtigkeit oder sogar einer anderen wässrigen Feuchtigkeit in Kontakt, so kann deren Funktionsfähigkeit für das spätere Messen einer Glukosekonzentration verschlechtert werden. Somit ist bei Verwendung derartiger trockenchemischer Testsubstanzen der erfindungsgemäß realisierte Feuchtigkeitsschutz besonders vorteilhaft.

Zum Beispiel kann die Testsubstanz auf einem Ring aus einem optisch transparenten Material aufgebracht sein, der über eine Kapillare im Inneren eines Gehäusekörpers der Bluttesteinheit in Fluidverbindung mit einer Position steht, an welcher die Stechhilfe (zum Beispiel eine Lanzette) in den Finger des Benutzers einsticht, um einen Bluttropfen zu entnehmen. Durch Wechselwirkung zwischen der Testsubstanz auf dem Ring einerseits und dem Bluttropfen andererseits kann eine chemische Reaktion ausgelöst werden, deren Reaktionsprodukt zum Beispiel optisch nachgewiesen werden kann. Damit diese Testsubstanz im Falle einer Blutzuckermessung funktioniert, muss diese vor dem erstmaligen Verwenden vor Feuchtigkeit zuverlässig geschützt werden, da die Feuchtigkeit die chemischen Eigenschaften der Testsubstanz vor Durchführung des Blutzuckertests unerwünscht verändern kann.

Gemäß einem Ausführungsbeispiel kann die Bluttesteinheit einen Gehäusekörper aufweisen, in dem die Stechhilfe versenkt ist. Die Bluttesteinheit kann ferner eine Fingerauflagefläche zum Auflegen eines Fingers des Benutzers derart aufweisen, dass bei Ausüben von Druck des Benutzers auf die Fingerauflagefläche die Stechhilfe durch eine Öffnung in der Fingerauflagefläche hindurch in den Finger eindringt, um das Blut zu entnehmen. Legt der Benutzer anschaulich seinen Finger auf die Fingerauflagefläche und übt Druck aus, so wird eine Fingerkuppe komprimiert und gleichzeitig das Herausfahren der Stechhilfe aus dem Gehäusekörper und in Richtung der komprimierten Fingerkuppe ausgelöst. Der Typ der einzelverpackten Bluttesteinheit kann derart sein, dass nach Aufsetzen der Bluttesteinheit auf das Detektorgerät das Ausüben von Druck durch einen Finger des Benutzers die Stechhilfe in Stechposition verfährt, so dass es zu einem Einstechen in den Finger und dem Fluss eines Bluttropfens durch eine Kapillare hin zu der Testsubstanz kommt. Um eine solche Bluttesteinheit erfindungsgemäß zu verwenden, ist es lediglich erforderlich, die Bluttesteinheit steril aus der Einzelverpackung zu entnehmen, auf das Detektorgerät aufzusetzen, mit dem Finger auf die Fingerauflagefläche zu drücken und das Ergebnis der Blutzuckermessung von einer Anzeige oder dergleichen des Detektorgeräts abzulesen.

Gemäß einem Ausführungsbeispiel kann der Gehäusekörper von einem klappbaren Deckel der Bluttesteinheit bedeckbar bzw. verschließbar sein, wobei der Deckel in einem in der Einzelverpackung verpackten Zustand der Bluttesteinheit die Fingerauflagefläche schützend überdeckt. Der Deckel ist ferner von einem Benutzer nach Herausnahme der Bluttesteinheit aus der Einzelverpackung zum Freilegen der Fingerauflagefläche klappbetätigbar. Im zugeklappten Zustand, in dem sich der Deckel befindet, wenn die Bluttesteinheit im Inneren der luftfeuchtigkeitsdichten Einzelverpackung untergebracht ist, bietet der Deckel einen zusätzlichen Schutz der empfindlichen inneren Komponenten der integralen Bluttesteinheit gegen Beschädigung oder unerwünschte Aktivierung der Stechhilfe vor dem Auspacken.

Gemäß einem Ausführungsbeispiel kann die Bluttesteinheit innerhalb der Einzelverpackung steril aufgenommen sein. Unter steril wird in diesem Zusammenhang das Freisein von vermehrungsfähigen Keimen, d.h. von Mikroorganismen und Viren, verstanden. Diese Sterilität kann durch das Durchführen eines Sterilisierungsverfahrens nach dem Unterbringen der Bluttesteinheit in der Einzelverpackung erreicht werden. Auf diese Weise können unerwünschte Infektionen eines Benutzers oder sonstige Beeinträchtigungen durch Verunreinigungen der Bluttesteinheit vermieden werden.

Gemäß einem Ausführungsbeispiel kann die luftfeuchtigkeitsdichte Einzelverpackung eine lokal begrenzte Materialschwächung, insbesondere eine Perforation oder Aufreißlinie, aufweisen, die bei händischer Betätigung durch einen Benutzer die Entnahme der Bluttesteinheit aus der Einzelverpackung in einer genau vordefinierten Weise ermöglicht. Eine derartige Aufrisshilfe kann es einem Benutzer ermöglichen, die integrale Bluttesteinheit derart aus der Einzelverpackung zu entnehmen, dass eine Beschädigung durch unsachgemäßes Öffnen vermieden ist. Zum Beispiel kann eine solche Materialschwächung durch selektives Entfernen (zum Beispiel mittels Stanzens oder Prägens) von Material einzelner Schichten einer Mehrschichtfolie derart erfolgen, dass das verbleibende Material zwar eine Luftfeuchtigkeitsdichtigkeit aufrechterhält, aber durch bloße Muskelkraft eines Benutzers aufgerissen werden kann. Insbesondere kann dadurch eine Aufreißtrajektorie vorgegeben werden.

Gemäß einem Ausführungsbeispiel kann die luftfeuchtigkeitsdichte Einzelverpackung eine mit sich selbst verschweißte oder heißversiegelte oder auch verklebte Mehrschichtstruktur (insbesondere die oben beschriebene Mehrschichtfolie) aufweisen. Somit kann die luftfeuchtigkeitsdichte Einzelverpackung aus einer - unter Ausformen eines nach außen hin luftfeuchtigkeitsdichten Innenvolumens - mit sich selbst verbundenen Mehrschichtfolie bestehen und von weiteren Komponenten frei sein. Dies erlaubt eine besonders kostengünstige Fertigung des Einweg-Produkts, ohne dass hinsichtlich der Wasserdichtigkeit Kompromisse eingegangen werden müssten.

Gemäß einem Ausführungsbeispiel kann bei dem Herstellungsverfahren das Innere der Einzelverpackung und somit die Bluttesteinheit nach dem hermetischen Umschließen durch die Einzelverpackung sterilisiert werden. Ein solches Sterilisieren kann insbesondere mittels Bestrahlens mit radioaktiver Strahlung (wie zum Beispiel Betastrahlung oder Gammastrahlung) erfolgen. Alternativ kann das Sterilisieren auch mittels einer Plasmabehandlung (zum Beispiel mittels eines Ozonplasmas, wobei Ozonpartikel durch die Einzelverpackung hindurchdringen können) erfolgen. Besonders vorteilhaft ist es, das Sterilisieren der Bluttesteinheit erst nach dem Verpackvorgang durchzuführen, da ein Sterilisieren vor Ausbilden der Einzelverpackung zu einem unerwünschten Eindringen von Fremdstoffen beim Ausbilden der Einzelverpackung führen kann, wodurch die Sterilität gefährdet werden kann.

Im Folgenden werden exemplarische Ausführungsbeispiele der vorliegenden Erfindung mit Verweis auf die folgenden Figuren detailliert beschrieben.
Figur 1 bis Figur 3 zeigen Querschnittsansichten von Einweg-Produkten gemäß exemplarischen Ausführungsbeispielen der Erfindung.
Figur 4 zeigt einen Trocknungsstreifen des Einweg-Produkts gemäß Figur 3.
Figur 5 zeigt eine Mehrschichtfolie einer Einzelverpackung eines Einweg-Produkts gemäß exemplarischen Ausführungsbeispielen der Erfindung.
Figur 6 und Figur 7 zeigen Bilder von Einweg-Produkten gemäß exemplarischen Ausführungsbeispielen der Erfindung.
Figur 8 zeigt eine integrale Bluttesteinheit eines Einweg-Produkts gemäß einem exemplarischen Ausführungsbeispiel Erfindung in einem Zustand mit geöffnetem Deckel.
Figur 9 zeigt eine Explosionsdarstellung der integralen Bluttesteinheit gemäß Figur 8.
Figur 10 zeigt eine Anordnung aus einer integralen Bluttesteinheit eines Einweg-Produkts sowie ein zugehöriges Detektorgerät gemäß einem exemplarischen Ausführungsbeispiel Erfindung.
Figur 11 zeigt ein Einweg-Produkt gemäß einem exemplarischen Ausführungsbeispiel der Erfindung, bei dem ein Teil einer Bluttesteinheit selbst Teil der Einwegverpackung bildet und bei dem Trocknungsmittel integral in der Bluttesteinheit vorgesehen ist.
Figur 12 zeigt eine Bluttesteinheit beim Einführen in einen becherförmigen Teil einer Einzelverpackung eines Produkts gemäß einem exemplarischen Ausführungsbeispiel der Erfindung.
Figur 13 zeigt eine räumliche oberseitige Ansicht der Anordnung aus Figur 12 nach dem Verschließen einer Öffnung des becherförmigen Teils mit einer Mehrschichtfolie als anderes Teil der Einzelverpackung.
Figur 14 zeigt eine räumliche unterseitige Ansicht der Anordnung aus Figur 13.
Figur 15 zeigt ein Einweg-Produkt gemäß einem exemplarischen Ausführungsbeispiel der Erfindung, bei dem Trocknungsmittel in eine Wandung einer Mehrschichtfolie als Einzelverpackung eingebettet ist.

Gleiche oder ähnliche Komponenten in unterschiedlichen Figuren sind mit gleichen Bezugsziffern versehen.

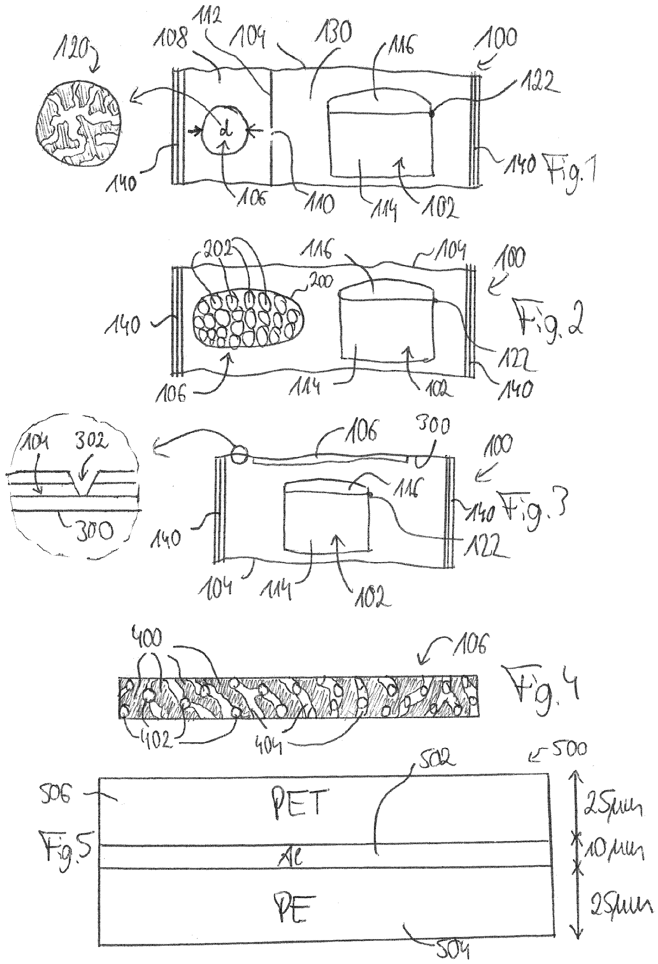

**Figur 1** zeigt eine Querschnittsansicht eines Einweg-Produkts 100 zum nur einmaligen Durchführen eines Bluttests durch einen Benutzer.

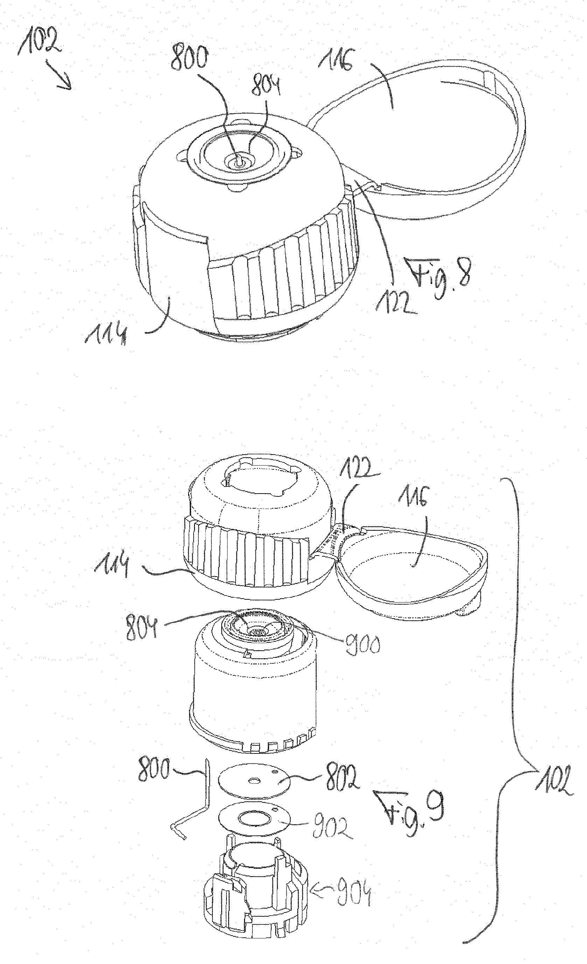

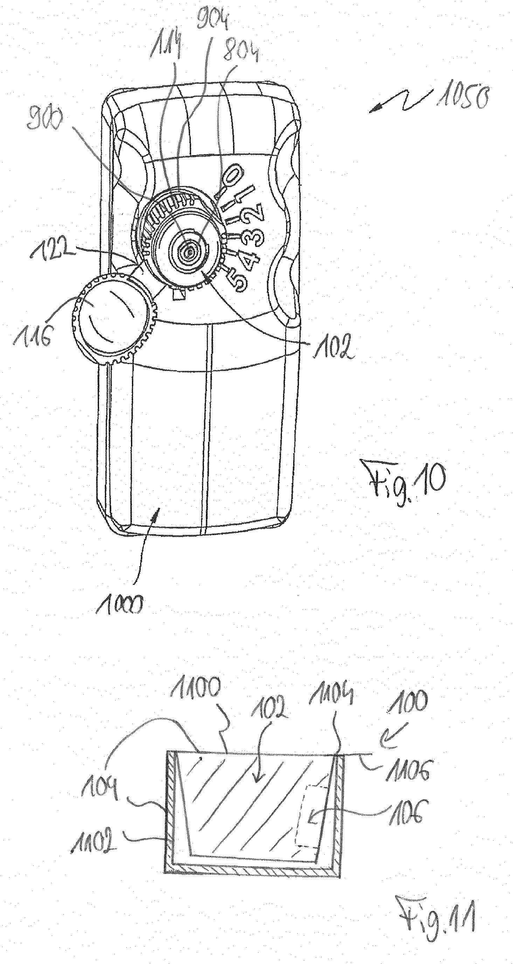

Das Einweg-Produkt 100 enthält eine integrale Bluttesteinheit 102, die in Figur 1 schematisch und von der Seite dargestellt ist. Für eine detailliertere Darstellung der Bluttesteinheit 102 wird auf Figur 8 und Figur 9 verwiesen. In der Ansicht des Einweg-Produkts 100 gemäß Figur 1, welche die Bluttesteinheit 102 vor der Benutzung im verpackten Zustand zeigt, ist ein Gehäusekörper 114 aus Kunststoff zu erkennen, der mittels eines Filmscharniers 122 mit einem klappbaren Deckel 116 verbunden ist. Im in Figur 1 gezeigten verpackten Zustand verschließt der Deckel 116 den Gehäusekörper 114 und schützt und verdeckt somit die darin enthaltenen funktionellen Komponenten. Eine Lanzette als Stechhilfe zum Einstechen in einen Finger des Benutzers zum Entnehmen einer Blutprobe ist somit im geschlossenen Zustand der Bluttesteinheit 102 ebenso wenig zu erkennen wie eine in der integralen Bluttesteinheit 102 vorgesehene trockenchemische Testsubstanz, die eine chemische Reaktion mit der entnommenen Blutprobe durchführen kann, um eine messtechnisch erfassbare und für einen Glukosespiegel des Benutzers indikative Menge eines Reaktionsprodukts zu erzeugen. Das Reaktionsprodukt kann optisch erfasst werden, wenn die Bluttesteinheit 102 auf einem Detektorgerät montiert ist, welches optische Komponenten zum Erfassen des Stoffs an der Position der trockenchemischen Testsubstanz aufweist. Ein solches Detektorgerät ist in Figur 10 gezeigt.

Die Testsubstanz ist außerordentlich empfindlich gegen Feuchtigkeit wie Wasserdampf oder Flüssigkeit wie Wasser. Selbst in der Gegenwart geringer Mengen von Feuchtigkeit reagiert die Testsubstanz mit derselben, was die spätere Benutzung der Bluttesteinheit 102 zum Durchführen eines Glukosetests negativ beeinträchtigt oder sogar ausschließt.

Um selbst bei langen Lagerzeiten der Bluttesteinheit 102 deren zuverlässige Entkopplung von wässrigen Medien aus der Umgebung sicherzustellen, enthält das Einwegprodukt 100 eine diese allseits umschließende wasserdichte Einzelverpackung 104 als Feuchtigkeitsbarriere, in welcher die Bluttesteinheit 102 eingeschlossen ist. Anders ausgedrückt umschließt die Einzelverpackung 104 die Bluttesteinheit 102 hermetisch. Hierfür ist die aus einer Mehrschichtfolie aufgebaute Einzelverpackung 104 an einzelnen Stellen mit sich selbst wasserdicht verschweißt, was schematisch in Form von Nahtstellen 140 dargestellt ist. Gleichzeitig ist aufgrund des Ausbildens der Einzelverpackung 104 als dünne Mehrschichtfolie die Einzelverpackung durch einen Benutzer durch Aufreißen händisch und werkzeugfrei öffenbar.

In dem verschlossenen und versiegelten Zustand des Einwegprodukts 100 gemäß Figur 1 befindet sich die integrale Bluttesteinheit 102 in einem sterilen Zustand. Dieser kann vorteilhaft ausgebildet werden, indem nach dem Umschließen der Bluttesteinheit 102 durch die Einzelverpackung 104 die so erzeugte, noch nicht notwendigerweise sterile Anordnung einem Sterilitätsverfahren unterzogen wird. Dieses kann darin bestehen, dass das noch nicht sterile Einweg-Produkt 100 einer geeigneten radioaktiven Strahlung ausgesetzt wird. Zum Beispiel kann Betastrahlung oder Gammastrahlung zu einer solchen Sterilisierung der Bluttesteinheit 102 nach dem Umgeben mit der fluiddichten Einzelverpackung 104 verwendet werden. Alternativ kann das noch nicht sterile Einweg-Produkt 100 mittels einer geeigneten Plasmabehandlung, zum Beispiel mittels Ozon Plasma, sterilisiert werden. Hierbei durchdringen anschaulich Plasmaionen die Einzelverpackung 104 und sterilisieren dadurch die Bluttesteinheit 102. diese Sterilisierungsverfahren sind mit der Bluttesteinheit 102 vorteilhaft durchführbar, da diese (mit Ausnahme einer kleinen metallischen Stechhilfe) aus Materialien wie Kunststoff besteht und geeignete Materialstärken hat, welche für das zu sterilisierende Medium (radioaktive Strahlung bzw. Ozon) ausreichend gut zugänglich sind. Die Effizienz dieser Sterilisierungsverfahren zum Sterilisieren der Bluttesteinheit 102 im bereits verpackten Zustand wird auch dadurch ermöglicht und gefördert, dass die Bluttesteinheit 102 innerhalb der Einzelverpackung 104 in Form einer dünnen Mehrschichtfolie aus geeigneten Materialien und mit geeigneten Dicken untergebracht ist, wie unten bezugnehmend auf Figur 5 näher beschrieben wird.

Wie ferner Figur 1 zu entnehmen ist, weist das Einweg-Produkt 100 ein Trocknungsmittel 106 in Form einer Kugel 120 mit einem Durchmesser von d=5 mm auf, die im Inneren der luftfeuchtigkeitsdichten Einzelverpackung 104 in Fluidverbindung mit der Bluttesteinheit 102 untergebracht ist. Das Trocknungsmittel 106 ist im gezeigten Ausführungsbeispiel als einzige Kugel 120 aus einem porösen Material wie zum Beispiel Molekularsieb oder Silikagel gebildet. Die hohe innere Oberfläche der Kugel 120, den Figur 1 auch im Detail dargestellt ist, bewirkt eine stark hygroskopische Wirkung, so dass eine etwaig durch die Mehrschichtfolie der Einzelverpackung 104 durchdringende Feuchtigkeit zuverlässig an der Kugel 120 gebunden wird, ohne auf die trockenchemische Testsubstanz der Bluttesteinheit 102 unerwünschte Wirkungen auszuüben.

Das Trocknungsmittel 106 ist gemäß dem Ausführungsbeispiel von Figur 1 somit in Form der Kugel 120 realisiert, die sich in einem separaten Kompartment 108 oder einer separaten Tasche der luftfeuchtigkeitsdichten Einzelverpackung 104 befindet. Das Kompartment 108 ist von einem anderen Kompartment 130, in dem sich die Bluttesteinheit 102 befindet, räumlich getrennt und durch ein Durchgangsloch 110 in einer Trennwand 112 in Fluidverbindung gebracht. Die Trennwand 112 verbindet zwei Hauptflächen der Einzelverpackung 104 miteinander. Das Kompartment 108 ist somit durch die Trennwand 112 von dem anderen Kompartment 130 der Einzelverpackung 104 getrennt, in dem die Bluttesteinheit 102 untergebracht ist. Alternativ könnten das Trocknungsmittel 106 und die Bluttesteinheit 102 in ein und demselben Kompartment angeordnet sein.

Wie an den diversen Nahtstellen 140 in Figur 1 zu erkennen ist, ist die Einzelverpackung 104 in Form der Verbundfolie mit sich selbst an mehreren Stellen verschweißt oder heißversiegelt, um dadurch die allseits hermetisch geschlossene feuchtigkeitsundurchlässige Einzelverpackung 104 zu bilden.

Ein in **Figur 2** gezeigtes Einweg-Produkt 100 gemäß einem anderen exemplarischen Ausführungsbeispiel der Erfindung unterscheidet sich von dem Ausführungsbeispiel gemäß Figur 1 insbesondere dadurch, dass das Trocknungsmittel 106 gemäß Figur 2 in Form von Granulat 202 aus Molekularsieb in einem feuchtigkeitsdurchlässigen, aber für das Granulat 202 undurchlässigen Sack 200 (zum Beispiel aus einem weitmaschigen Gewebe oder einer feuchtigkeitsdurchlässigen Wandung) im Inneren der luftfeuchtigkeitsdichten Einzelverpackung 104 ausgebildet ist. Sowohl das Trocknungsmittel 106 als auch die Bluttesteinheit 102 sind gemäß Figur 2 in einem gemeinsamen Kompartment der Einzelverpackung 104 angeordnet. Alternativ könnten das Trocknungsmittel 106 und die Bluttesteinheit 102, ähnlich wie in Figur 1 gezeigt, in unterschiedlichen Kompartments angeordnet sein.

Ein in **Figur 3** gezeigtes Einweg-Produkt 100 gemäß noch einem anderen exemplarischen Ausführungsbeispiel der Erfindung unterscheidet sich von den Ausführungsbeispielen gemäß Figur 1 und Figur 2 insbesondere dadurch, dass gemäß Figur 3 die beiden äußeren Schichten der hier dreischichtigen Verbundfolie, welche die Einzelverpackung 104 bildet, lokal mittels Stanzens oder Prägens entfernt sind, womit eine in einem Detail in Figur 3 im Querschnitt gezeigte Aufreißlinie 302 gebildet ist. Wenn ein Benutzer die Einzelverpackung 104 an der Aufreißlinie 302 händisch aufreißt, so ist ein definiertes Öffnen entlang der Aufreißlinie 302 und somit ein Entnehmen der Bluttesteinheit 102 mit geringem Kraftaufwand ermöglicht.

Ferner ist das Trocknungsmittel 106 gemäß Figur 3 als Trocknungsstreifen auf einer Innenwand 300 der luftfeuchtigkeitsdichten Einzelverpackung 104 heißlaminiert (hierbei verschmilzt die bei Zimmertemperatur feste Innenwand 300 der Einzelverpackung 104 durch thermische Behandlung mit dem Trocknungsstreifen) oder kaltlaminiert (hierbei befindet sich zwischen der Innenwand 300 der Einzelverpackung 104 und dem Trocknungsstreifen Klebstoff, der in Figur 3 nicht gezeigt ist).

**Figur 4** zeigt den Aufbau des Trocknungsstreifens gemäß Figur 3 im Querschnitt. Das eigentliche Trocknungsmittel 106 liegt hier in Form von Trocknungspartikeln 402 vor, die in einer porösen Matrix 400 aus festem Kunststoff angeordnet sind. Durch die feuchtigkeitsdurchlässigen Poren 404 sind die zum Beispiel aus Molekularsieb hergestellten Trocknungspartikel 402 mit dem Innenraum der Einzelverpackung 104 fluidisch gekoppelt und können daher möglicherweise im Innenraum befindliche Feuchtigkeit aufnehmen. Hierbei dienen die engen Kanäle 404 dazu, die feuchtigkeitsbindende Wirkung der Trocknungspartikel 402 gezielt abzuschwächen, um die feuchtigkeitsbindende Wirkung über einen langen Zeitraum hinweg aufrechterhalten zu können. Dies ist zum Erreichen langer Lagerzeiten vorteilhaft. Das Kompositmaterial des Trocknungsstreifens gemäß Figur 4 kann hergestellt werden, indem Kunststoff und die Trocknungspartikel 402 vermischt werden, wobei der verwendete Kunststoff so ausgewählt wird, dass sich das Kunststoffmaterial mit den Trocknungspartikeln 402 nicht oder nur schlecht vermischt. Genau dadurch entsteht die Kanalstruktur.

**Figur 5** zeigt einen Querschnitt einer Mehrschichtfolie 500 oder Verbundfolie, wie sie zum Bilden der Einzelverpackung 104, zum Beispiel in den Ausführungsbeispielen gemäß Figur 1 bis Figur 3, vorteilhaft eingesetzt werden kann.

Kern der Mehrschichtfolie 500 ist eine Feuchtigkeitsdiffusionsbarrierenschicht 502, die als 10 µm dicke Aluminiumschicht ausgebildet ist. Diese hat die Funktion, ein Durchdringen von Feuchtigkeit oder sogar Wasser durch die Mehrschichtfolie 500 zu unterbinden. Hierfür ist Aluminium hervorragend geeignet.

Die Feuchtigkeitsdiffusionsbarrierenschicht 502 ist auf einer heißversiegelbaren Schicht 504 aufgebracht, die in dem gezeigten Ausführungsbeispiel als 25 µm dicke Polyethylenschicht ausgebildet ist. Diese übernimmt zum einen die Aufgabe, als Substrat zum Tragen und Aufbringen der Aluminiumschicht zu dienen. Eine weitere wichtige Funktion der heißversiegelbaren Schicht 504 ist deren Verwendung zum Heißversiegeln mit sich selbst, um die Nahtstellen 140 und somit die hermetisch abgeschlossene Einzelverpackung 104 auszubilden, die auch an den heißversiegelten Nahtstellen 140 luftfeuchtigkeitsdicht ist. Hierfür ist Polyethylen besonders gut geeignet. Bei dem Einweg-Produkt 100 bildet die heißversiegelbare Schicht 504 die Innenschicht.

Auf einer gegenüberliegenden Hauptoberfläche der Feuchtigkeitsdiffusionsbarrierenschicht 502 ist eine Schutzschicht 506 vorgesehen, die im gezeigten Ausführungsbeispiel als 25 µm dicke Polyethylenterephthalat-Schicht (PET) ausgebildet ist. Diese schützt die Feuchtigkeitsdiffusionssperrschicht 502 vor externen mechanischen Einflüssen und kann mit einer beliebigen Aufschrift bedruckt werden (zum Beispiel kann eine Charge oder eine Identifikationsnummer der Bluttesteinheit 102 darauf abgedruckt sein, womit die Authentizität der Bluttesteinheit 102 sichergestellt werden kann). Bei dem Einweg-Produkt 100 bildet die Schutzschicht 506 die Außenschicht.

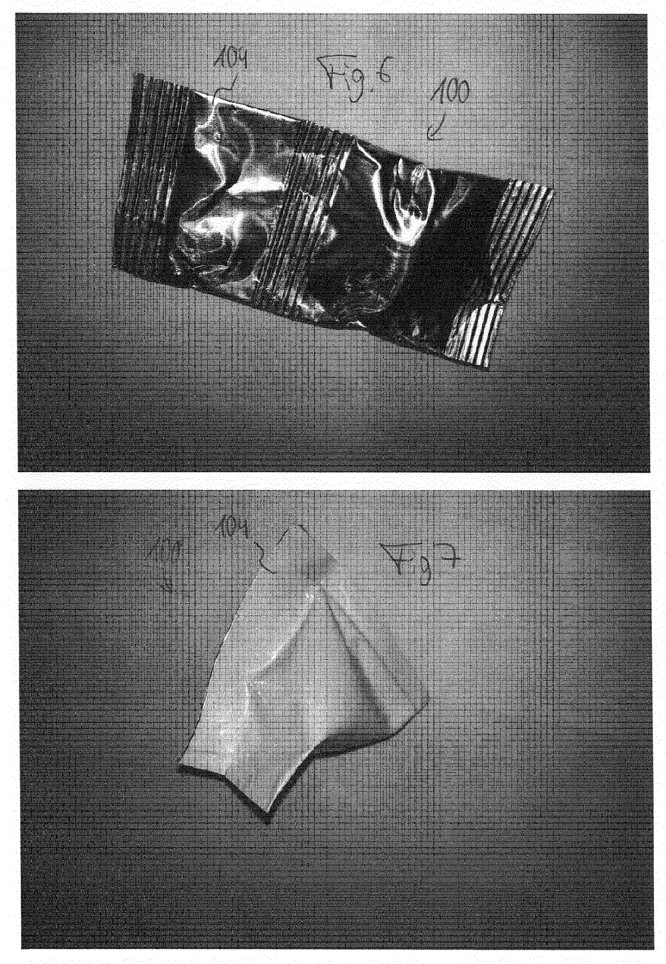

**Figur 6** zeigt ein Bild eines Einweg-Produkts 100 gemäß einem exemplarischen Ausführungsbeispiel der Erfindung, bei dem die Schutzschicht durchsichtig ist, so dass die darunter gelegene Metallschicht als Feuchtigkeitsdiffusionssperre zu erkennen ist. Die Einzelverpackung 104 weist gemäß Figur 6 eine schlauchartige Geometrie auf, wobei mittels Schweißens unterschiedliche Schlauchabschnitte definiert sind.

**Figur 7** zeigt ein Bild eines Einweg-Produkts 100 gemäß einem anderen exemplarischen Ausführungsbeispiel der Erfindung, bei dem die Schutzschicht aus einem undurchsichtigen Kunststoffmaterial gebildet ist, so dass die darunter gelegene Metallschicht als Feuchtigkeitsdiffusionssperre nicht zu erkennen ist. Die Einzelverpackung 104 hat gemäß Figur 7 eine annähernd tetraedrische Form.

**Figur 8** ist eine genauere Darstellung der Bluttesteinheit 102 in einem Betriebszustand, in dem der Deckel 116 mittels des Filmscharniers 122 gegenüber dem Gehäusekörper 114 aufgeklappt ist.

Durch das Aufklappen des Deckels 116 wird eine Fingerauflagefläche 804 zum Auflegen eines Fingers des Benutzers freigelegt. Wie Figur 8 zu entnehmen ist, ist die Fingerauflagefläche 804 durch konzentrische Ringe mit einer dazwischen gebildeten diskontinuierlichen Übergangskante ausgebildet. Legt ein Benutzer seinen Finger auf die Fingerauflagefläche 804, so wird ein zentraler Abschnitt der Fingerkuppe innerhalb der Übergangs kannte durch diese zusammengepresst, womit ein nachfolgendes Einstechen einer zunächst noch in dem Gehäusekörper 114 versenkten Stechhilfe 800 erleichtert wird. Die Stechhilfe 800 ist in Figur 8 bereits gezeigt und durchdringt dort ein Durchgangsloch der Fingerauflagefläche 804.

Nach dem Entnehmen der Bluttesteinheit 102 aus der Einzelverpackung 104 und nach dem Öffnen des Deckels 116 gegenüber dem Gehäusekörper 114 ist die Stechhilfe 800 allerdings noch im Inneren des Gehäusekörpers 114 angeordnet. Erst nach Aufsetzen der Bluttesteinheit 102 auf ein Detektorgerät (siehe Figur 10) und nachfolgendes Ausüben von Druck mit dem Finger auf die Fingerauflagefläche 804 wird die Stechhilfe 800 aus dem Gehäusekörper 114 herausgefahren und sticht dadurch automatisch in den aufgelegten Finger ein. Dadurch wird im Bereich der Fingerauflagefläche 804 ein Bluttropfen aus dem Finger entnommen. Dieser fließt durch eine Kapillare, die direkt neben der Stechhilfe vertikal nach unten verläuft, in Richtung der Testsubstanz, wodurch an der Testsubstanz eine chemische Reaktion zwischen dem Blut des Benutzers und der Testsubstanz ausgelöst wird. Ein chemisches Reaktionsprodukt kann dann optisch nachgewiesen werden, wofür eine Lichtquelle und ein Lichtdetektor des Detektorgeräts gemäß Figur 10 verwendet werden können.

Figur 8 zeigt, dass die Länge, die Breite und die Höhe der Bluttesteinheit 102 sich voneinander nur in geringem Umfang unterscheiden, womit die Bluttesteinheit 102 als sehr kompakter Körper ausgebildet ist.

**Figur 9** ist eine Explosionsdarstellung der Bluttesteinheit 102 gemäß Figur 8. Figur 9 sind eine Reihe von Einzelkomponenten zu entnehmen, die in Figur 8 nicht zu erkennen sind.

Ein unterseitiges Adapterstück 904 dient zum Aufsetzen auf das Detektorgerät gemäß Figur 10. Ein darüber angeordnetes Kalibrationsplättchen 902 kann optisch lesbare Kalibrationsinformation, zum Beispiel in Form von Farbreferenzen und/oder Barcodes, enthalten. Diese kann das Detektorgerät auslesen, um eine Glukosemessung zu kalibrieren. Über dem Kalibrationsplättchen 902 ist ein transparenter Ring angeordnet, der die Testsubstanz 802 enthält. Ist die Bluttesteinheit 102 auf dem Detektorgerät montiert, so kann das Detektorgerät von unten her mittels Durchführens einer optischen Messung Eigenschaften der Testsubstanz 802 erfassen, die sich infolge der Wechselwirkung mit dem Blut verändert haben. Hieraus kann dann das Detektorgerät die Glukosekonzentration im Blut des Benutzers bestimmen. Oberhalb des Rings, der die Testsubstanz 802 enthält, ist ein weiterer Körper angeordnet, in dem die Kapillare 900 verläuft und an dessen Oberseite die Fingerauflagefläche 804 gebildet sind. Die gemäß Figur 9 oberste Komponente enthält den Gehäusekörper 114, das Filmscharnier 122 und den Deckel 116.

Wichtig ist noch zu erwähnen, dass die Bluttesteinheit 102 eine Mechanik hat, bei der nach dem erstmaligen Einsatz der Stechhilfe 800 ein nochmaliger Einsatz der Stechhilfe 800 verunmöglicht ist. Dies wird dadurch erreicht, dass nach dem Einstechen der Stechhilfe 800 in einen Finger diese nachfolgend irreversibel ins Innere des Gehäusekörpers 114 zurück gezogen und dort gelagert wird und daher nicht ein weiteres Mal nach oben aus der Fingerauflagefläche 804 austreten kann.

**Figur 10** zeigt eine Anordnung 1050, bei der eine Bluttesteinheit 102 mit den oben beschriebenen Merkmalen direkt nach dem Entnehmen aus der Einwegverpackung 104 auf ein Detektorgerät 1000 aufgesetzt worden ist. Das Detektorgerät 1000 kann zum Beispiel ein Handheldgerät oder ein Mobilfunkgerät sein, das entsprechend auf ein Zusammenwirken mit der Bluttesteinheit 102 angepasst ist. Das Detektorgerät 1000 enthält eine Aufnahmeaussparung, die konfiguriert ist, die Bluttesteinheit 102 an deren Unterseite 904 formschlüssig aufzunehmen. Nach Öffnen des Deckels 116 braucht ein Benutzer dann bloß noch mit etwas Druck seinen Finger auf die Fingerauflagefläche 804 aufzulegen, wodurch die Stechhilfe 800 aus dem Gehäusekörper 114 heraustritt, um dadurch dem aufgelegten Finger des Benutzers einen Blutstropfen entnehmen. Dieser fließt durch die Kapillare 900 hindurch direkt auf die Testsubstanz 802. Eine Lichtquelle (nicht gezeigt) des Detektorgeräts 1000 emittiert dann Licht in Richtung der Testsubstanz 802. Nach Wechselwirkung des chemischen Reaktionsprodukts aus Testsubstanz 802 und Blutstropfen mit dem eingestrahlten Licht wird Sekundärlicht in Richtung eines Lichtdetektors (nicht gezeigt) des Detektorgeräts 1000 zurückgestrahlt. Dies kann zum Beispiel Fluoreszenzlicht oder reflektiertes Licht sein. Aus dem sekundären Licht kann das Detektorgerät 1000 dann die Glukosekonzentration im Blut bestimmen. Die Bluttesteinheit 102 wird dann von dem Detektorgerät 1000 abgezogen und ohne nochmalige Verwendung entsorgt.

**Figur 11** zeigt ein Einweg-Produkt 100 gemäß einem exemplarischen Ausführungsbeispiel der Erfindung, bei dem ein gemäß Figur 11 oberseitiger Oberflächenbereich 1100 einer Bluttesteinheit 102 selbst Teil der Einwegverpackung 104 bildet und bei dem Trocknungsmittel 106 integral in der Bluttesteinheit 102 vorgesehen ist. Anders ausgedrückt bilden ein becherförmiges Teil 1102 und der Oberflächenbereich 1100 selbst gemeinsam die Einwegverpackung 104. Ein Übergangs- oder Nahtbereich 1104 zwischen dem becherförmigen Teil 1102 und dem Oberflächenbereich 1100 der Bluttesteinheit 102 ist feuchtigkeitsdicht auszubilden, zum Beispiel mittels einer händisch zu öffnenden Klebe- oder Siegelverbindung. Zum Beispiel kann eine Abziehlasche 1106 einstückig mit der Bluttesteinheit 102 ausgebildet werden, so dass ein Benutzer bei Abziehen der Abziehlasche 1106 die Bluttesteinheit 102 von dem becherförmigen Teil 1102 lösen kann.

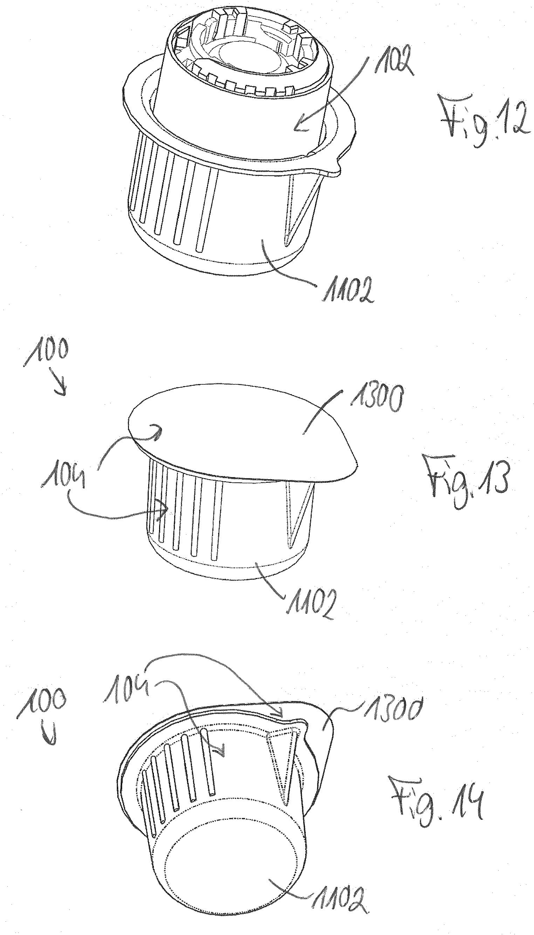

**Figur 12** zeigt eine Bluttesteinheit 102 beim Einführen in einen becherförmigen Teil 1102 einer Einzelverpackung 104 eines Produkts 100 gemäß einem exemplarischen Ausführungsbeispiel der Erfindung.

**Figur 13** zeigt eine räumliche oberseitige Ansicht der Anordnung aus Figur 12 nach dem Verschließen einer Öffnung des becherförmigen Teils 1102 mit einer Mehrschichtfolie 1300 als anderes Teil der Einzelverpackung 104.

**Figur 14** zeigt eine räumliche unterseitige Ansicht der Anordnung aus Figur 13.

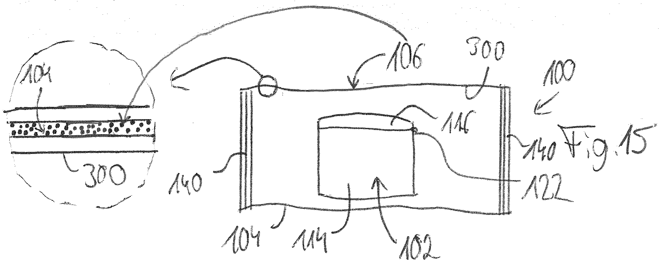

**Figur 15** zeigt ein Einweg-Produkt 100 gemäß einem exemplarischen Ausführungsbeispiel der Erfindung, bei dem Trocknungsmittel 106 in eine Wandung einer Mehrschichtfolie 300 als Einzelverpackung eingebettet ist. Somit ist das Trocknungsmittel 106 in der als Feuchtigkeitsbarriere wirkenden Einzelverpackung selbst eingebettet bzw integriert bzw. ist als Teil einer beidseitig bedeckten Mittelschicht der Mehrschichtfolie 300 ausgebildet. Dann kann Flüssigkeit bereits während des Durchdringens der Einzelverpackung 104 gebunden werden und gelangt somit gar nicht erst zur Bluttesteinheit 102, sondern wird anschaulich zuvor abgefangen.

Ergänzend ist darauf hinzuweisen, dass "aufweisend" keine anderen Elemente oder Schritte ausschließt und "eine" oder "ein" keine Vielzahl ausschließt. Ferner sei darauf hingewiesen, dass Merkmale oder Schritte, die mit Verweis auf eines der obigen Ausführungsbeispiele beschrieben worden sind, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

## Patentansprüche

1. Einweg-Produkt (100) zum einmaligen Durchführen eines Bluttests durch einen Benutzer, wobei das Einweg-Produkt (100) aufweist:
eine integrale Bluttesteinheit (102) mit einer Stechhilfe (800) zum Einstechen in ein Körperteil des Benutzers zum Entnehmen einer Blutprobe und mit einer Testsubstanz (802) zum Wechselwirken mit der entnommenen Blutprobe zum Erzeugen eines detektierbaren Indikators für eine Eigenschaft der Blutprobe, wenn die Bluttesteinheit (102) mit einem Detektorgerät (1000) gekoppelt ist; und
eine Einzelverpackung (104) als Feuchtigkeitsbarriere, welche zumindest einen Teil der Bluttesteinheit (102) gegenüber einer Umgebung hermetisch umschließt und die von einem Benutzer händisch öffenbar ist,
wobei die Einzelverpackung (104) eine Mehrschichtfolie (500) aufweist, die eine Feuchtigkeitsdiffusionsbarrierenschicht (502) enthält, deren eine Oberfläche mit einer heißversiegelbaren Schicht (504) bedeckt ist und deren gegenüberliegende andere Oberfläche mit einer Schutzschicht (506) zum Schutz der Feuchtigkeitsdiffusionsbarrierenschicht (502) bedeckt ist,
wobei die Einzelverpackung (104) einen starren Grundkörper (1102), insbesondere ausgebildet als Spritzgussteil, aufweist, der mit der Mehrschichtfolie (500) verschlossen ist, und
wobei der starre Grundkörper (1102) becherförmig ist und eine Öffnung aufweist, die von der Mehrschichtfolie (500) ringförmig umschlossen ist.

2. Produkt (100) gemäß Anspruch 1, wobei die Einzelverpackung (104) im Wesentlichen flüssigkeitsdicht, insbesondere luftfeuchtigkeitsdicht, weiter insbesondere gasdicht ist.

3. Produkt (100) gemäß Anspruch 1, wobei die Einzelverpackung (104) ausschließlich aus der Mehrschichtfolie (500) besteht.

4. Produkt (100) gemäß einem der Ansprüche 1 bis 3, wobei die Feuchtigkeitsdiffusionsbarrierenschicht (502) ein Metall, insbesondere Aluminium oder Nickel aufweist oder daraus besteht.

5. Produkt (100) gemäß einem der Ansprüche 1 bis 4, wobei die Feuchtigkeitsdiffusionsbarrierenschicht (502) ein Metalloxid, Ethylenvinylacetat oder ein Silikat aufweist oder daraus besteht.

6. Produkt (100) gemäß einem der Ansprüche 1 bis 5, wobei die heißversiegelbare Schicht (504) einen heißversiegelbaren Kunststoff, insbesondere Polyethylen oder Polypropylen, aufweist oder daraus besteht.

7. Produkt (100) gemäß einem der Ansprüche 1 bis 6, wobei die Schutzschicht (506) Polyamid, Polyethylenterephthalat, eine Polyethylenterephthalat-Papier Struktur oder Papier aufweist oder daraus besteht.

8. Produkt (100) gemäß einem der Ansprüche 1 bis 7, wobei die als Feuchtigkeitsbarriere wirkende Einzelverpackung (104) nur genau einen Teil der Bluttesteinheit (102) umschließt und ein verbleibender Teil der Bluttesteinheit (102) gegenüber der Umgebung unbedeckt bleibt und ebenfalls als Feuchtigkeitsbarriere wirkend ausgebildet ist.

9. Produkt (100) gemäß einem der Ansprüche 1 bis 8, aufweisend ein Trocknungsmittel (106), das in Flüssigkeitsverbindung, insbesondere in Feuchtigkeitsverbindung, mit der Bluttesteinheit (102) angeordnet ist.

10. Produkt (100) gemäß Anspruch 9, wobei das Trocknungsmittel (106) im Inneren der als Feuchtigkeitsbarriere wirkenden Einzelverpackung (104) angeordnet ist.

11. Produkt (100) gemäß Anspruch 9, wobei das Trocknungsmittel (106) in der als Feuchtigkeitsbarriere wirkenden Einzelverpackung (104) eingebettet oder aufgebracht ist.

12. Produkt (100) gemäß einem der Ansprüche 9 bis 11, wobei das Trocknungsmittel (106) ein Molekularsieb, Silikagel und/oder Tonerde aufweist.

13. Produkt (100) gemäß Anspruch 9, 10 oder 12, wobei das Trocknungsmittel (106), insbesondere in Form von Granulat (202) oder Pulver, in einem feuchtigkeitsdurchlässigen und trocknungsmittelundurchlässigem Sack (200) im Inneren der als Feuchtigkeitsbarriere wirkenden Einzelverpackung (104) angeordnet ist.

14. Produkt (100) gemäß Anspruch 9, 10, 12 oder 13, wobei das Trocknungsmittel (106), insbesondere in Form eines einzigen zusammenhängenden Körpers (120), in einem separaten Kompartment (108) der als Feuchtigkeitsbarriere wirkenden Einzelverpackung (104) angeordnet ist, das mit der Bluttesteinheit (102), insbesondere durch ein Durchgangsloch (110) in einer Wand (112) des Kompartments (108), in Flüssigkeitsverbindung, insbesondere in Feuchtigkeitsverbindung, gebracht ist.

15. Produkt (100) gemäß Anspruch 9, 10 oder 12 bis 14, wobei das Trocknungsmittel (106), insbesondere in Form von in einer porösen Matrix (400) verteilten Trocknungsmittelpartikeln (402), als flächige Struktur auf einer Innenwand (300) der als Feuchtigkeitsbarriere wirkenden Einzelverpackung (104) befestigt ist, insbesondere laminiert oder aufgeklebt.

16. Produkt (100) gemäß einem der Ansprüche 9, 10 oder 12 bis 15, wobei das Trocknungsmittel (106) an und/oder in der Bluttesteinheit (102) mechanisch verankert ist oder ein Trockenmittelcompound als struktureller Bestandteil der Bluttesteinheit (102) vorgesehen ist.

17. Produkt (100) gemäß einem der Ansprüche 1 bis 16, wobei innerhalb der Einzelverpackung (104) nur genau die eine Bluttesteinheit (102) enthalten ist.

18. Produkt (100) gemäß einem der Ansprüche 1 bis 17, wobei die Bluttesteinheit (102) derart ausgebildet ist, dass bei dieser nach erstmaligem Einsatz der Stechhilfe (800) ein nochmaliger Einsatz der Stechhilfe (800) verunmöglicht ist.

19. Produkt (100) gemäß einem der Ansprüche 1 bis 18, wobei die Testsubstanz (802) eine trockenchemische, durch Feuchtigkeit veränderliche Substanz aufweist.

20. Produkt (100) gemäß einem der Ansprüche 1 bis 19, wobei die Bluttesteinheit (102) einen Gehäusekörper (114) aufweist, in dem die Stechhilfe (800) vor Durchführung des Bluttests versenkt ist, und wobei die Bluttesteinheit (102) eine Fingerauflagefläche (804) zum Auflegen eines Fingers des Benutzers derart aufweist, dass bei Ausüben von Druck des Benutzers auf die Fingerauflagefläche (804) die Stechhilfe (800) durch eine Öffnung in der Fingerauflagefläche (804) hindurch in den Finger eindringt, um das Blut zu entnehmen.

21. Produkt (100) gemäß Anspruch 20, wobei der Gehäusekörper (114) von einem klappbaren Deckel (116) bedeckbar ist, der in einem in der Einzelverpackung (104) verpackten Zustand der Bluttesteinheit (102) die Fingerauflagefläche (804) überdeckt und der von einem Benutzer nach Herausnahme der Bluttesteinheit (102) aus der Einzelverpackung (104) zum Freilegen der Fingerauflagefläche (804) klappbetätigbar ist.

22. Produkt (100) gemäß einem der Ansprüche 1 bis 21, wobei die Bluttesteinheit (102) innerhalb der Einzelverpackung (104) steril aufgenommen ist.

23. Produkt (100) gemäß einem der Ansprüche 1 bis 22, wobei die als Feuchtigkeitsbarriere wirkende Einzelverpackung (104) eine lokal begrenzte Materialschwächung (302), insbesondere eine Perforation oder Aufreißlinie, aufweist, die bei händischer Betätigung durch einen Benutzer die Entnahme der Bluttesteinheit (102) aus der Einzelverpackung (104) ermöglicht.

24. Produkt (100) gemäß einem der Ansprüche 1 bis 23, wobei die als Feuchtigkeitsbarriere wirkende Einzelverpackung (104) eine mit sich selbst verschweißte oder heißversiegelte Mehrschichtstruktur (500) aufweist.

25. Test-Anordnung, aufweisend:
ein Einweg-Produkt (100) gemäß einem der Ansprüche 1 bis 24 zum einmaligen Durchführen eines Bluttests durch einen Benutzer;
ein Detektorgerät (1000), mit dem die Bluttesteinheit (102) derart koppelbar ist, dass im gekoppelten Zustand die Stechhilfe (800) der Bluttesteinheit (102) in ein Körperteil des Benutzers zum Entnehmen einer Blutprobe einstechbar ist und die entnommene Blutprobe mit der Testsubstanz (802) der Bluttesteinheit (102) zum Erzeugen eines Indikators für eine Eigenschaft der Blutprobe in Wechselwirkung bringbar ist, welcher Indikator mittels des Detektorgeräts (1000) detektierbar ist.

26. Verfahren zum Herstellen eines Einweg-Produkts (100) zum einmaligen Durchführen eines Bluttests durch einen Benutzer, wobei das Verfahren aufweist:
Bereitstellen einer integralen Bluttesteinheit (102) mit einer Stechhilfe (800) zum Einstechen in ein Körperteil des Benutzers zum Entnehmen einer Blutprobe und mit einer Testsubstanz (802) zum Wechselwirken mit der entnommenen Blutprobe zum Erzeugen eines für eine Eigenschaft der Blutprobe indikativen Indikators, der detektierbar ist, wenn die Bluttesteinheit (102) mit einem Detektorgerät (1000) gekoppelt ist; und
hermetisches Umschließen zumindest eines Teils der Bluttesteinheit (102) gegenüber einer Umgebung mit einer Einzelverpackung (104) als Feuchtigkeitsbarriere derart, dass die Einzelverpackung (104) von einem Benutzer händisch öffenbar ist, wobei die Einzelverpackung (104) eine Mehrschichtfolie (500) aufweist, die eine Feuchtigkeitsdiffusionsbarrierenschicht (502) enthält, deren eine Oberfläche mit einer heißversiegelbaren Schicht (504) bedeckt ist und deren gegenüberliegende andere Oberfläche mit einer Schutzschicht (506) zum Schutz der Feuchtigkeitsdiffusionsbarrierenschicht (502) bedeckt ist,
wobei die Einzelverpackung (104) einen starren Grundkörper (1102), insbesondere ausgebildet als Spritzgussteil, aufweist, der mit der Mehrschichtfolie (500) verschlossen ist, und
wobei der starre Grundkörper (1102) becherförmig ist und eine Öffnung aufweist, die von der Mehrschichtfolie (500) ringförmig umschlossen ist.

27. Verfahren gemäß Anspruch 26, ferner aufweisend Sterilisieren, insbesondere Sterilisieren mittels Bestrahlens mit radioaktiver Strahlung oder mittels einer Plasmabehandlung, der Bluttesteinheit (102) nach dem hermetischen Umschließen durch die Einzelverpackung (104).

## Claims

1. A single-use product (100) for performing a blood test by a user once only, wherein the single-use product (100) comprises:
an integral blood test unit (102) having a piercing aid (800) for piercing into a body part of the user for taking a blood sample, and having a test substance (802) for interacting with the blood sample taken for producing a detectable indicator of a characteristic of the blood sample when the blood test unit (102) is coupled to a detector device (1000); and
an individual packaging (104) as a moisture barrier, which hermetically encloses at least a part of the blood test unit (102) relative to an environment and which may be opened manually by a user,
wherein the individual packaging (104) comprises a multi-layer film (500) containing a moisture diffusion barrier layer (502), one surface of which is covered by a hot-sealable layer (504) and the opposite other surface of which is covered by a protective layer (506) for protecting the moisture diffusion barrier layer (502),
wherein the individual packaging (104) comprises a rigid basic body (1102), in particular implemented as an ejection molding part, which is sealed by the multi-layer film (500), and
wherein the rigid basic body (1102) is cup-shaped and comprises an opening which is annularly enclosed by the multi-layer film (500).

2. The product (100) in accordance with claim 1, wherein the individual packaging (104) is essentially liquid-tight, in particular humidity-tight, and in particular gas-tight.

3. The product (100) in accordance with claim 1, wherein the individual packaging (104) consists exclusively of the multi-layer film (500).

4. The product (100) in accordance with any of claims 1 to 3, wherein the moisture diffusion barrier layer (502) comprises a metal, in particular aluminum or nickel, or is made thereof.

5. The product (100) in accordance with any of claims 1 to 4, wherein the moisture diffusion barrier layer (502) comprises a metal oxide, ethylene vinyl acetate or a silicate, or is made thereof.

6. The product (100) in accordance with any of claims 1 to 5, wherein the hot-sealable layer (504) comprises a hot-sealable plastic, in particular polyethylene or polypropylene, or is made thereof.

7. The product (100) in accordance with any of claims 1 to 6, wherein the protective layer (506) comprises polyamide, polyethylene terephthalate, a polyethylene terephthalate paper structure or paper, or is made thereof.

8. The product (100) in accordance with any of claims 1 to 7, wherein the individual packaging (104) acting as a moisture barrier encloses only precisely one part of the blood test unit (102) and a remaining part of the blood test unit (102) remains uncovered relative to the environment and is also implemented so as to act as a moisture barrier.

9. The product (100) in accordance with any of claims 1 to 8, comprising a drying agent (106) which is arranged in liquid connection, in particular in moisture connection, to the blood test unit (102).

10. The product (100) in accordance with claim 9, wherein the drying agent (106) is arranged inside the individual packaging (104) acting as a moisture barrier.

11. The product (100) in accordance with claim 9, wherein the drying agent (106) is embedded or applied in the individual packaging (104) acting as a moisture barrier.

12. The product (100) in accordance with any of claims 9 to 11, wherein the drying agent (106) comprises a molecular sieve, silica gel and/or alumina.

13. The product (100) in accordance with any of claims 9, 10 or 12, wherein the drying agent (106), in particular in the form of a granulate (202) or powder, is arranged in a moisture-permeable and drying agent-impermeable bag (200) inside the individual packaging (104) acting as a moisture barrier.

14. The product (100) in accordance with any of claims 9, 10, 12 or 13, wherein the drying agent (106), in particular in the form of a single contiguous body (120), is arranged in a separate compartment (106) of the individual packaging (104) acting as a moisture barrier, which is brought into liquid connection, in particular moisture connection, to the blood test unit (102), in particular through a via hole (110) in a wall (112) of the compartment (108).

15. The product (100) in accordance with any of claims 9, 10 or 12 to 14, wherein the drying agent (106), in particular in the form of drying agent particles (402) distributed in a porous matrix (400), is attached, in particular laminated or glued, as a two-dimensional structure, on an inside wall (300) of the individual packaging (104) acting as a moisture barrier.

16. The product (100) in accordance with any of claims 9, 10 or 12 to 15, wherein the drying agent (106) is mechanically anchored on and/or in the blood test unit (102) or a drying agent compound is provided as a structural element of the blood test unit (102).

17. The product (100) in accordance with any of claims 1 to 16, wherein only the precisely one blood test unit (102) is contained within the individual packaging (104).

18. The product (100) in accordance with any of claims 1 to 17, wherein the blood test unit (102) is configured such that, after having used the piercing aid (801) once, making use of the piercing aid (800) again is made impossible.

19. The product (100) in accordance with any of claims 1 to 18, wherein the test substance (802) comprises a dry-chemical substance alterable by moisture.

20. The product (100) in accordance with any of claims 1 to 19, wherein the blood test unit (102) comprises a casing body (114) where the piercing aid (800) is sunk into before performing the blood test, and wherein the blood test unit (102) comprises a finger support area (804) for placing a finger of the user thereon such that, when the user applies a pressure on the finger support area (804), the piercing aid (800) penetrates into the finger through an opening in the finger support area (804) in order to take blood.

21. The product (100) in accordance with claim 20, wherein the casing body (114) is coverable by a foldable lid (116) which, in a state of the blood test unit (102) packaged in the individual packaging (104), covers the finger support area (804) and which is folded and, thus, actuated by a user upon taking the blood test unit (102) from the individual packaging (104), for exposing the finger support area (804).

22. The product (100) in accordance with any of claims 1 to 21, wherein the blood test unit (102) is received within the individual packaging (104) in a sterile manner.

23. The product (100) in accordance with any of claims 1 to 22, wherein the individual packaging (104) acting as a moisture barrier comprises a locally limited material weakening (302), in particular a perforation or tear-up line, which, when operated manually by a user, allows taking the blood test unit (102) from the individual packaging (104).

24. The product (100) in accordance with any of claims 1 to 23, wherein the individual packaging (104) acting as a moisture barrier comprises a multi-layer structure (500) sealed to itself or a hot-sealed multi-layer structure (500).

25. A test arrangement comprising:
a single-use product (100) in accordance with any of claims 1 to 24 for performing a blood test by a user once only;
a detector device (1000) which may be coupled to the blood test unit (102) such that, in the coupled state, the piercing aid (800) of the blood test unit (102) may be pierced into a body part of the user for taking a blood sample and the blood sample taken may be brought into interaction with the test substance (802) of the blood test unit (102) for producing an indicator of a characteristic of the blood sample, said indicator being detectable by means of the detector device (1000).

26. A method for producing a single-use product (100) for performing a blood test by a user once only, wherein the method comprises:
providing an integral blood test unit (102) having a piercing aid (800) for piercing into a body part of the user for taking a blood sample, and having a test substance (802) for interacting with the blood sample taken for producing an indicator indicative of a characteristic of the blood sample, which is detectable when the blood test unit (102) is coupled to a detector device (1000); and
hermetically enclosing at least a part of the blood test unit (102) relative to an environment using an individual packaging (104) as a moisture barrier such that the individual packaging (104) may be opened manually by a user,
wherein the individual packaging (104) comprises a multi-layer film (500) containing a moisture diffusion barrier layer (502), one surface of which is covered by a hot-sealable layer (502) and the opposite other layer of which is covered by a protective layer (506) for protecting the moisture diffusion barrier layer (502),
wherein the individual packaging (104) comprises a rigid basic body (1102), in particular implemented as an injection molding part, which is sealed by the multi-layer film (500), and
wherein the rigid basic body (1102) is cup-shaped and comprises an opening which is annularly enclosed by the multi-layer film (500).

27. The method in accordance with claim 26, further comprising sterilizing, in particular sterilizing by means of irradiating by radioactive radiation or by means of plasma treatment, the blood test unit (102) after hermetically enclosing the same by the individual packaging (104).

## Revendications

1. Produit à usage unique (100) pour une réalisation unique d'un test sanguin par un utilisateur, le produit à usage unique (100) présentant:
une unité de test sanguin intégrale (102) avec un moyen de percée (800) destiné à être introduit dans une partie de corps de l'utilisateur pour prélever un échantillon de sang et avec une substance de test (802) destinée à interagir avec l'échantillon de sang prélevé pour générer un indicateur d'une propriété de l'échantillon de sang détectable lorsque l'unité de test sanguin (102) est couplée à un appareil de détection (1000); et
un emballage unique (104) comme barrière à l'humidité qui entoure hermétiquement au moins une partie de l'unité de test sanguin (102) pour la protéger d'un environnement et qui peut être ouvert manuellement par un utilisateur;
dans lequel l'emballage unique (104) présente un film multicouche (500) contenant une couche barrière contre la diffusion de l'humidité (502) dont une surface est recouverte d'une couche thermoscellable (504) et dont l'autre surface opposée est recouverte d'une couche protectrice (506) destinée à protéger la couche barrière contre la diffusion de l'humidité (502),
dans lequel l'emballage unique (104) présente un corps de base rigide (1102), en particulier réalisé sous forme de pièce moulée par injection, qui est obturé par le film multicouche (500), et
dans lequel le corps de base rigide (1102) est en forme de coupe et présente une ouverture qui est entourée en forme d'anneau par le film multicouche (500).

2. Produit (100) selon la revendication 1, dans lequel l'emballage unique (104) est sensiblement étanche aux liquides, en particulier étanche à l'humidité dans l'air, par ailleurs en particulier étanche aux gaz.

3. Produit (100) selon la revendication 1, dans lequel l'emballage unique (104) est constitué exclusivement du film multicouche (500).

4. Produit (100) selon l'une des revendications 1 à 3, dans lequel la couche barrière contre la diffusion d'humidité (502) présente un métal, en particulier de l'aluminium ou du nickel, ou est réalisée dans ce dernier.

5. Produit (100) selon l'une des revendications 1 à 4, dans lequel la couche barrière contre la diffusion de l'humidité (502) présente un oxyde métallique, de l'acétate d'éthylène et de vinyle ou un silicate, ou est réalisée dans ce dernier.

6. Produit (100) selon l'une des revendications 1 à 5, dans lequel la couche thermoscellable (504) présente un matériau plastique thermoscellable, en particulier du polyéthylène ou du polypropylène, ou est réalisée dans ce dernier.

7. Produit (100) selon l'une des revendications 1 à 6, dans lequel la couche de protection (506) présente du polyamide, du téréphtalate de polyéthylène, une structure de papier ou du papier à base de téréphtalate de polyéthylène, ou est réalisée dans ce dernier.

8. Produit (100) selon l'une des revendications 1 à 7, dans lequel l'emballage unique agissant comme barrière contre l'humidité (104) n'entoure précisément qu'une partie de l'unité de test sanguin (102) et une partie restante de l'unité de test sanguin (102) reste découverte par rapport à l'environnement et est également réalisée comme barrière contre l'humidité.

9. Produit (100) selon l'une des revendications 1 à 8, présentant un agent de séchage (106) qui est disposé en communication de liquide, en particulier en communication d'humidité, avec l'unité de test sanguin (102).

10. Produit (100) selon la revendication 9, dans lequel l'agent de séchage (106) est disposé à l'intérieur de l'emballage unique agissant comme barrière contre l'humidité (104).

11. Produit (100) selon la revendication 9, dans lequel l'agent de séchage (106) est incorporé dans ou déposé sur l'emballage unique agissant comme barrière contre l'humidité (104).

12. Produit (100) selon l'une des revendications 9 à 11, dans lequel l'agent de séchage (106) présente un tamis moléculaire, un gel de silice et/ou de l'alumine.

13. Produit (100) selon la revendication 9, 10 ou 12, dans lequel l'agent de séchage (106), en particulier sous forme de granulés (202) ou de poudre, dans un sac (200) perméable à l'humidité et imperméable à l'agent de séchage est disposé à l'intérieur de l'emballage unique (104) agissant comme barrière contre l'humidité.

14. Produit (100) selon la revendication 9, 10, 12 ou 13, dans lequel l'agent de séchage (106), en particulier sous forme d'un corps cohérent unique (120), est disposé dans un compartiment séparé (108) de l'emballage unique agissant comme barrière contre l'humidité (104) qui est amené en communication de liquide, en particulier en communication d'humidité, avec l'unité de test sanguin (102), en particulier à travers un trou traversant (110) dans une paroi (112) du compartiment (108).

15. Produit (100) selon la revendication 9, 10 ou 12 à 14, dans lequel l'agent de séchage (106), en particulier sous forme de particules d'agent de séchage (402) réparties dans une matrice poreuse (400), est fixé, en particulier laminé ou collé, comme structure plane sur une paroi intérieure (300) de l'emballage unique (104) agissant comme barrière contre l'humidité.

16. Produit (100) selon l'une des revendication 9, 10 ou 12 à 15, dans lequel l'agent de séchage (106) est ancré mécaniquement sur et/ou dans l'unité de test sanguin (102) ou un composé de l'agent de séchage est prévu comme composant structurel de l'unité de test sanguin (102).

17. Produit (100) selon l'une des revendications 1 à 16, dans lequel dans l'emballage unique (104) n'est contenue qu'exactement une unité de test sanguin (102).

18. Produit (100) selon l'une des revendications 1 à 17, dans lequel l'unité de test sanguin (102) est conçue de sorte que dans celle-ci, après la première utilisation du moyen de percée (800), une nouvelle utilisation du moyen de percée (800) soit impossible.

19. Produit (100) selon l'une des revendications 1 à 18, dans lequel la substance de test (802) présente une substance chimique sèche variable par l'humidité.

20. Produit (100) selon l'une des revendications 1 à 19, dans lequel l'unité de test sanguin (102) présente un corps de boîtier (114) dans lequel est enfoui le moyen de percée (800) avant la réalisation du test sanguin, et dans lequel l'unité de test sanguin (102) présente une surface d'appui de doigt (804) destinée à placer un doigt de l'utilisateur de sorte que, lors de l'exercice d'une pression par l'utilisateur sur la surface d'appui de doigt (804), le moyen de percée (800) pénètre à travers une ouverture dans la surface d'appui de doigt (804) dans le doigt pour prélever le sang.

21. Produit (100) selon la revendication 20, dans lequel le corps de boîtier (114) est recouvert par un couvercle rabattable (116) qui recouvre, dans un état emballé de l'unité de test sanguin (102) dans l'emballage unique (104), la surface d'appui de doigt (804) et qui peut être actionné en rabattement par l'utilisateur après retrait de l'unité de test sanguin (102) de l'emballage unique (104) en vue d'exposer la surface d'appui de doigt (804).

22. Produit (100) selon l'une des revendications 1 à 21, dans lequel l'unité de test sanguin (102) est reçue stérilisée à l'intérieur de l'emballage unique (104).

23. Produit (100) selon l'une des revendications 1 à 22, dans lequel l'emballage unique (104) agissant comme barrière contre l'humidité présente un affaiblissement du matériau limité localement (302), en particulier une ligne de perforation ou de déchirure, qui, lors de l'actionnement manuel par un utilisateur, permet le retrait de l'unité de test sanguin (102) de l'emballage unique (104).

24. Produit (100) selon l'une des revendications 1 à 23, dans lequel l'emballage unique agissant comme barrière contre l'humidité (104) présente une structure multicouche auto-soudée ou thermoscellée (500).

25. Aménagement de test, présentant:
un produit à usage unique (100) selon l'une des revendications 1 à 24 pour la réalisation unique d'un test sanguin par un utilisateur;
un appareil de détection (1000) auquel l'unité de test sanguin (102) peut être couplée de sorte que, à l'état couplé, le moyen de percée (800) de l'unité de test sanguin (102) puisse être introduit dans une partie de corps de l'utilisateur pour le prélèvement d'un échantillon de sang, et l'échantillon de sang prélevé peut être amené en interaction avec la substance de test (802) de l'unité de test sanguin (102) pour générer un indicateur d'une propriété de l'échantillon de sang, indicateur qui est détectable au moyen de l'appareil de détection (1000).

26. Procédé de fabrication d'un produit à usage unique (100) pour une réalisation unique d'un test sanguine par un utilisateur, le procédé présentant le fait de:
prévoir une unité de test sanguin intégrale (102) avec un moyen de percée (800) destiné à être introduit dans une partie de corps de l'utilisateur pour prélever un échantillon de sang et avec une substance de test (802) destinée à interagir avec l'échantillon de sang prélevé pour générer un indicateur d'une propriété de l'échantillon de sang qui est détectable lorsque l'unité de test sanguin (102) est couplée à un appareil de détection (1000); et
entourer hermétiquement au moins une partie de l'unité de test sanguin (102), pour la protéger contre un environnement, d'un emballage unique (104) comme barrière contre l'humidité de sorte que l'emballage unique (104) puisse être ouvert manuellement par un utilisateur,
dans lequel l'emballage unique (104) présente un film multicouche (500) qui contient une couche barrière contre la diffusion de l'humidité (502) dont une surface est recouverte par une couche thermoscellable (504) et dont l'autre surface opposée est recouverte d'une couche de protection (506) destinée à protéger la couche barrière contre la diffusion de l'humidité (502),
dans lequel l'emballage unique (104) présente un corps de base rigide (1102), en particulier réalisé sous forme de pièce moulée par injection, qui est obturé par le film multicouche (500), et
dans lequel le corps de base rigide (1102) est en forme de coupe et présente une ouverture qui est entourée en forme d'anneau par le film multicouche (500).

27. Procédé selon la revendication 26, présentant, par ailleurs, le fait de stériliser, en particulier de stériliser au moyen d'une irradiation par rayonnement radioactif ou au moyen d'un traitement au plasma, l'unité de test sanguin (102) après l'entourage hermétique par l'emballage unique (104).
